(19) Europäisches Patentamt
European Patent Office
Office européen des brevets

(11) Veröffentlichungsnummer : **0 688 832 A2**

(12) # EUROPÄISCHE PATENTANMELDUNG

(21) Anmeldenummer : **95810343.4**

(22) Anmeldetag : **23.05.95**

(51) Int. Cl.[6] : **C09B 62/507,** C09B 62/08, C09B 67/22

(30) Priorität : **30.05.94 CH 1679/94**

(43) Veröffentlichungstag der Anmeldung : **27.12.95 Patentblatt 95/52**

(84) Benannte Vertragsstaaten : **BE CH DE ES FR GB IT LI PT**

(71) Anmelder : **CIBA-GEIGY AG**
**Klybeckstrasse 141**
**CH-4002 Basel (CH)**

(72) Erfinder : **Herzig, Paul**
**Ackerstrasse 52**
**CH-4057 Basel (CH)**
Erfinder : **Andreoli, Anton**
**Im Hirshalm 58**
**CH-4125 Riehen (CH)**

(54) **Reaktivfarbstoffe, ihre Herstellung und Verwendung**

(57) Es werden Verbindungen der Formel

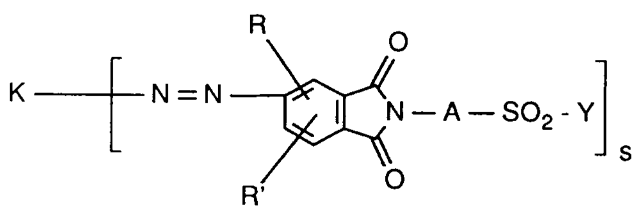

(1),

worin die Variablen die in den Ansprüchen angegebene Bedeutung haben, beschrieben, welche als faserreaktive Farbstoffe zum Färben oder Bedrucken von verschiedensten Fasermaterialien geeignet sind.

EP 0 688 832 A2

Die vorliegende Erfindung betrifft neue Reaktivfarbstoffe, Verfahren zu ihrer Herstellung und ihre Verwendung.

Die Praxis des Färbens mit Reaktivfarbstoffen hat in neuerer Zeit zu erhöhten Anforderungen an die Qualität der Färbungen und die Wirtschaftlichkeit des Färbeprozesses geführt. Infolge dessen besteht weiterhin ein Bedarf nach neuen Reaktivfarbstoffen, welche verbesserte Eigenschaften, insbesondere in bezug auf die Applikation, aufweisen.

Für das Färben werden heute Reaktivfarbstoffe gefordert, die eine ausreichende Substantivität haben und die zugleich eine gute Auswaschbarkeit der nicht fixierten Anteile aufweisen. Sie sollen ferner eine gute färberische Ausbeute aufweisen und hohe Reaktivität besitzen, wobei insbesondere Färbungen mit hohen Fixiergraden geliefert werden sollen. Von den bekannten Farbstoffen werden diese Anforderungen nicht in allen Eigenschaften erfüllt.

Der vorliegenden Erfindung liegt daher die Aufgabe zugrunde, neue, verbesserte Reaktivfarbstoffe für das Färben und Bedrucken von Fasermaterialien zu finden, welche die oben charakterisierten Qualitäten in hohem Masse besitzen. Die neuen Farbstoffe sollten sich vor allem durch hohe Fixierausbeuten und hohe Faser-Farbstoff-Bindungsstabilitäten auszeichnen, und ausserdem sollten die nicht auf der Faser fixierten Anteile leicht auswaschbar sein. Sie sollten ferner Färbungen mit guten Allgemeinechtheiten, beispielsweise Licht- und Nassechtheiten, ergeben.

Es hat sich gezeigt, dass mit den weiter unten definierten neuen reaktiven Farbstoffen die gestellte Aufgabe weitgehend gelöst wird.

Gegenstand der vorliegenden Erfindung sind Verbindungen der Formel

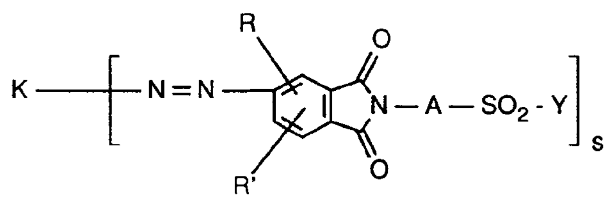 (1),

worin
K der Rest einer Kupplungskomponente ist,
s für die Zahl 1 oder 2 steht,
R und R' unabhängig voneinander je Wasserstoff, Sulfo, Hydroxy, $C_1$-$C_4$-Alkoxy, Halogen oder Cyano bedeuten,
A unsubstituiertes oder durch Halogen, Hydroxy, Sulfato, Carboxy, Cyano, $C_2$-$C_4$-Alkanoyloxy, $C_1$-$C_4$-Alkoxycarbonyl oder Carbamoyl substituiertes oder durch eine Gruppe -O- oder -NR''- unterbrochenes $C_1$-$C_6$-Alkylen oder unsubstituiertes oder im Phenylenteil durch $C_1$-$C_4$-Alkyl, $C_1$-$C_4$-Alkoxy, Halogen oder Sulfo substituiertes $C_1$-$C_6$-Alkylenphenylen ist,
R'' für Wasserstoff oder $C_1$-$C_4$-Alkyl steht,
Y Vinyl oder einen Rest $-CH_2-CH_2-U$ bedeutet, und
U eine alkalisch abspaltbare Abgangsgruppe ist.

Der Begriff Sulfo umfasst hierbei generell sowohl die freie Säure $-SO_3H$ als auch eine beliebige Salzform, z.B. ein Alkali-, Erdalkali- oder Ammoniumsalz oder das Salz eines organischen Amins wie etwa das Natrium-, Kalium-, Lithium- oder Ammoniumsalz oder das Salz des Triethanolamins.

$C_1$-$C_4$-Alkyl bedeutet generell Methyl, Ethyl, n- oder iso-Propyl oder n-, iso-, sec.- oder tert.-Butyl. $C_1$-$C_4$-Alkoxy bedeutet generell Methoxy, Ethoxy, n- oder iso-Propoxy oder n-, iso-, sec.- oder tert.-Butoxy und vorzugsweise Methoxy oder Ethoxy. Halogen steht generell z.B. für Brom oder Chlor. Beispiele für $C_2$-$C_4$-Alkanoyloxy sind Acetyl oder Propionyl, Beispiele für $C_1$-$C_4$-Alkoxycarbonyl sind Methoxycarbonyl oder Ethoxycarbonyl. Unter $C_1$-$C_6$-Alkylen ist generell geradkettiges oder in beliebiger Weise verzweigtes $C_1$-$C_6$-Alkylen zu verstehen; Beispiele sind Methylen, 1,1- oder 1,2-Ethylen, 1,2- oder 1,3-Propylen, 1,2-, 1,3-, 1,4- oder 2,3-Butylen, 1,4-, 2,3- oder 2,4-Pentylen, 2-Methyl-1,5-pentylen und 1,6-Hexylen, wobei diese Reste wie angegeben substituiert oder mit Ausnahme von Methylen durch ein Heteroatom -O- oder -NR''- unterbrochen sein können.

R und R' bedeuten unabhängig voneinander bevorzugt Wasserstoff, Sulfo, Chlor, Brom, Hydroxy, Methoxy oder Cyano; besonders bevorzugt steht R für Wasserstoff, Sulfo, Chlor, Brom oder Cyano und R' für Wasserstoff; insbesondere bevorzugt bedeuten R Wasserstoff oder Sulfo und R' Wasserstoff. Eine besonders bevorzugte Ausführungsform der Erfindung betrifft Verbindungen der Formel (1), worin R und R' je Wasserstoff sind.

R'' bedeutet vorzugsweise Wasserstoff, Methyl oder Ethyl und besonders bevorzugt Wasserstoff.

Bedeutet A einen $C_1$-$C_6$-Alkylenphenylenrest, handelt es sich bevorzugt um unsubstituiertes oder im Phenylenteil durch Methyl, Methoxy, Chlor oder Sulfo substituiertes Methylenphenylen oder Ethylenphenylen und besonders bevorzugt um Methylenphenylen.

A bedeutet vorzugsweise einen unsubstituierten $C_1$-$C_6$-Alkylenrest oder einen Rest der Formel

$$-(CH_2)_p-O-(CH_2)_q-$$

oder

$$-(CH_2)_p-NH-(CH_2)_q-,$$

worin p und q unabhängig voneinander je eine ganze Zahl von 1 bis 6 darstellen.

A steht besonders bevorzugt für einen $C_2$-$C_4$-Alkylenrest oder für einen Rest der Formel

$$-(CH_2)_{2-4}-O-(CH_2)_{2-4}-$$

oder

$$-(CH_2)_{2-4}-NH-(CH_2)_{2-4}-.$$

Insbesonders bevorzugte Bedeutungen von A sind 1,2-Ethylen, 1,3-Propylen, 1,4-Butylen oder ein Rest der Formel

$$-(CH_2)_{2-3}-O-(CH_2)_{2-3}-$$

oder

$$-(CH_2)_{2-3}-NH-(CH_2)_{2-3}-.$$

Eine besonders bevorzugte Ausführungsform der vorliegenden Erfindung betrifft Verbindungen der Formel (1), worin A 1,2-Ethylen, 1,3-Propylen oder einen Rest der Formel $-(CH_2)_2-O-(CH_2)_2-$ bedeutet.

Geeignete alkalisch abspaltbare Abgangsgruppen U sind z.B. Halogen wie etwa Chlor oder Brom, Acyloxy wie etwa Acetoxy oder Benzoyloxy, Phosphato, Sulfato oder Thiosulfato.

Beispiele für geeignete Reste Y sind dementsprechend Vinyl, β-Brom- oder β-Chlorethyl, β-Acetoxyethyl, β-Benzoyloxyethyl, β-Phosphatoethyl, β-Sulfatoethyl und β-Thiosulfatoethyl. Y steht bevorzugt für Vinyl oder β-Sulfatoethyl.

Von besonderem Interesse sind Verbindungen der zuvor angegebenen Formel (1), worin R Wasserstoff, Sulfo, Chlor, Brom oder Cyano; R' Wasserstoff; A unsubstituiertes $C_1$-$C_6$-Alkylen oder einen Rest der Formel

$$-(CH_2)_p-O-(CH_2)_q-$$

oder

$$-(CH_2)_p-NH-(CH_2)_q-,$$

worin p und q unabhängig voneinander je eine ganze Zahl von 1 bis 6 darstellen; und Y Vinyl, β-Brom- oder β-Chlorethyl, β-Acetoxyethyl, β-Benzoyloxyethyl, β-Phosphatoethyl, β-Sulfatoethyl oder β-Thiosulfatoethyl bedeuten.

Von ganz besonderem Interesse sind Verbindungen der zuvor angegebenen Formel (1), worin R Wasserstoff oder Sulfo; R' Wasserstoff; A $C_2$-$C_4$-Alkylen oder einen Rest der Formel $-(CH_2)_{2-4}-O-(CH_2)_{2-4}-$ oder $-(CH_2)_{2-4}-NH-(CH_2)_{2-4}-$; und Y Vinyl oder β-Sulfatoethyl bedeuten.

Eine insbesondere bevorzugte Ausführungsform der vorliegenden Erfindung stellen Verbindungen der zuvor angegebenen Formel (1) dar, worin R und R' je Wasserstoff, A 1,2-Ethylen, 1,3-Propylen oder einen Rest der Formel $-(CH_2)_2-O-(CH_2)_2-$ und Y β-Sulfatoethyl bedeuten.

K bedeutet z.B. den Rest einer Kupplungskomponente der Benzol-, Naphthalin-, Naphthylazobenzol-, Phenylazonaphthalin-, 4-Alkyl-6-hydroxypyridon-(2)-, 2,5-Diamino-4-alkylpyridin-, 1-Arylpyrazolon-(5)- oder 1-Aryl-5-aminopyrazol-Reihe, wobei diese farbstoffübliche Substituenten und gegebenenfalls eine oder mehrere faserreaktive Gruppen enthalten kann.

Aus der Reihe der Substituenten seien beispielhaft genannt: Alkylgruppen mit 1 bis 12 Kohlenstoffatomen, insbesondere 1 bis 4 Kohlenstoffatomen, wie Methyl, Ethyl, n- oder iso-Propyl, oder n-, iso-, sec.- oder tert.-Butyl, Alkoxygruppen mit 1 bis 8 Kohlenstoffatomen, insbesondere 1 bis 4 Kohlenstoffatomen, wie Methoxy, Ethoxy, n-oder iso-Propoxy, oder n-, iso-, sec.- oder tert.-Butoxy, im Alkylteil z.B. durch Hydroxy, $C_1$-$C_4$-Alkoxy oder Sulfato substituiertes $C_1$-$C_4$-Alkoxy wie z.B. 2-Hydroxyethoxy, 3-Hydroxypropoxy, 2-Sulfatoethoxy, 2-Methoxyethoxy oder 2-Ethoxyethoxy, Acylaminogruppen mit 2 bis 8 Kohlenstoffatomen, insbesondere $C_2$-$C_4$-Alkanoylaminogruppen wie Acetylamino oder Propionylamino, Benzoylamino oder $C_2$-$C_4$-Alkoxycarbonylaminogruppen wie Methoxycarbonylamino oder Ethoxycarbonylamino, Amino, gegebenenfalls im Alkylteil z.B. durch Hydroxy, Sulfo, Sulfato oder $C_1$-$C_4$-Alkoxy substituiertes N-Mono- oder N,N-Di-$C_1$-$C_4$-Alkylamino, z.B. Methylamino, Ethylamino, N,N-Dimethyl- oder N,N-Diethylamino, Sulfomethylamino, β-Hydroxyethylamino, N,N-Di-(2-hydroxyethylamino), N-β-Sulfatoethylamino, gegebenenfalls im Phenylteil durch Methyl, Methoxy, Halogen oder Sulfo substituiertes Phenylamino, gegebenenfalls im Alkylteil durch Hydroxy, Sulfo oder Sulfato oder im Phenylteil gegebenenfalls durch Methyl, Methoxy, Halogen oder Sulfo substituiertes N-$C_1$-$C_4$-Alkyl-N-phenylamino, z.B. N-Methyl-N-phenylamino, N-Ethyl-N-phenylamino, N-β-Hydroxyethyl-N-phenylamino oder N-β-Sulfoethyl-N-phenylamino, gegebenenfalls durch Sulfo substituiertes Naphthylamino,

Alkanoylgruppen mit 2 bis 8 Kohlenstoffatomen, insbesondere 2 bis 4 Kohlenstoffatomen, z.B. Acetyl oder Propionyl, Benzoyl, Alkoxycarbonyl mit 1 bis 4 Kohlenstoffatomen im Alkoxyrest, wie Methoxycarbonyl oder Ethoxycarbonyl, Alkylsulfonyl mit 1 bis 4 Kohlenstoffatomen, wie Methylsulfonyl oder Ethylsulfonyl, Phenyl- oder Naphthylsulfonyl, Trifluormethyl, Nitro, Cyano, Hydroxyl, Halogen, wie Fluor, Chlor oder Brom, Carbamoyl, N-$C_1$-$C_4$-Alkylcarbamoyl, wie N-Methylcarbamoyl oder N-Ethylcarbamoyl, Sulfamoyl, N-$C_1$-$C_4$-Alkylsulfamoyl wie N-Methylsulfamoyl, N-Ethylsulfamoyl, N-Propylsulfamoyl, N-Isopropylsulfamoyl oder N-Butylsulfamoyl, N-(β-Hydroxyethyl)-sulfamoyl, N,N-Di-(β-hydroxyethyl)-sulfamoyl, N-Phenylsulfamoyl, Ureido, Carboxy, Sulfomethyl, Sulfo oder Sulfato sowie faserreaktive Reste . Die Alkylreste können zudem durch Sauerstoff (-O-) unterbrochen sein.

Unter faserreaktiven Resten sind solche zu verstehen, die mit den Hydroxygruppen der Cellulose, den Amino-, Carboxy-, Hydroxy- und Thiolgruppen bei Wolle und Seide, oder mit den Amino- und eventuell Carboxygruppen von synthetischen Polyamiden unter Bildung kovalenter chemischer Bindungen zu reagieren vermögen. Die faserreaktiven Reste sind in der Regel direkt oder über ein Brückenglied an den Farbstoffrest gebunden. Geeignete faserreaktive Reste sind beispielsweise solche, die mindestens einen abspaltbaren Substituenten an einem aliphatischen, aromatischen oder heterocyclischen Rest enthalten oder worin die genannten Reste einen zur Reaktion mit dem Fasermaterial geeigneten Rest, wie z.B. einen Vinylrest, enthalten.

Enthält K einen faserreaktiven Rest, so entspricht dieser bevorzugt der Formel

$-SO_2-Y$ (2a),

$-CONR_1-(CH_2)_n-SO_2-Y$ (2b)

oder

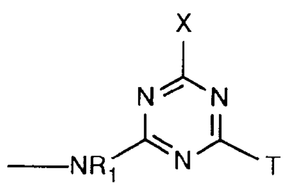

(2c),

worin X Halogen, 3-Carboxypyridin-1-yl oder 3-Carbamoylpyridin-1-yl bedeutet, T unabhängig die Bedeutung von X hat oder für Hydroxy, $C_1$-$C_4$-Alkoxy, Phenoxy, $C_1$-$C_4$-Alkylthio, Morpholino, gegebenenfalls durch nichtreaktive Reste substituiertes Amino oder für einen Reaktivrest der Formel

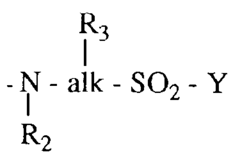

(3a),

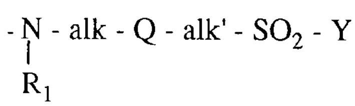

(3b),

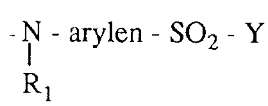

(3c),

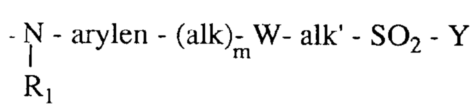

(3d),

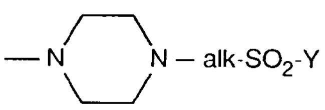

(3e)

oder

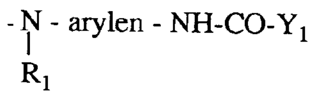

(3f)

steht,
$R_1$ Wasserstoff oder $C_1$-$C_4$-Alkyl ist,
$R_2$ Wasserstoff, gegebenenfalls durch Hydroxy, Sulfo, Sulfato, Carboxy oder Cyano substituiertes $C_1$-$C_4$-Alkyl oder einen Rest

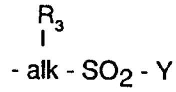

bedeutet,
$R_3$ Wasserstoff, Hydroxy, Sulfo, Sulfato, Carboxy, Cyano, Halogen,
$C_1$-$C_4$-Alkoxycarbonyl, $C_1$-$C_4$-Alkanoyloxy, Carbamoyl oder die Gruppe $-SO_2$-Y ist, alk und alk' unabhängig voneinander $C_1$-$C_6$-Alkylen sind,
arylen einen unsubstituierten oder durch Sulfo, Carboxy, $C_1$-$C_4$-Alkyl, $C_1$-$C_4$-Alkoxy oder Halogen substituierten Phenylen- oder Naphthylenrest bedeutet,
$Y_1$ für eine Gruppe $-CHX_2-CH_2X_2$ oder $-CX_2=CH_2$ steht und $X_2$ Chlor oder Brom bedeutet,
Q ein Rest -O- oder -NR''-,worin R'' die zuvor angegebene Bedeutung hat, ist,
W für eine Gruppe $-SO_2-NR_2-$, $-CONR_2-$ oder $-NR_2CO-$ steht,
m die Zahl 0 oder 1 ist,
n für eine ganze Zahl von 1 bis 6 steht und für Y unabhängig die zuvor genannte Bedeutung gilt.

Die Variable $R_1$ steht bevorzugt für Wasserstoff, Methyl oder Ethyl und besonders bevorzugt für Wasserstoff.

Die Variable n bedeutet bevorzugt die Zahl 1, 2, 3 oder 4, besonders bevorzugt die Zahl 2 oder 3 und insbesondere bevorzugt die Zahl 2.

Steht T für gegebenenfalls durch nicht-reaktive Reste substituiertes Amino, so kann es sich um Amino, N-$C_1$-$C_4$-Alkylamino oder N,N-Di-$C_1$-$C_4$-Alkylamino, wobei das Alkyl gegebenenfalls z.B. durch Sulfo, Sulfato, Hydroxy, Carboxy oder Phenyl substituiert ist, Cyclohexylamino, N-$C_1$-$C_4$-Alkyl-N-phenylamino oder Phenylamino oder Naphthylamino, wobei das Phenyl oder Naphthyl gegebenenfalls z.B. durch $C_1$-$C_4$-Alkyl, $C_1$-$C_4$-Alkoxy, $C_2$-$C_4$-Alkanoylamino, Carboxy, Sulfo oder Halogen substituiert ist, handeln.

Beispiele für geeignete nicht-reaktive Reste T sind Amino, Methylamino, Ethylamino, β-Hydroxyethylamino, N,N-Di-β-Hydroxyethylamino, β-Sulfoethylamino, Cyclohexylamino, Morpholino, o-, m- oder p-Chlorphenylamino, o-, m- oder p-Methylphenylamino, o-, m- oder p-Methoxyphenylamino, o-, m- oder p-Sulfophenylamino, Disulfophenylamino, o-Carboxyphenylamino, 1- oder 2-Naphthylamino, 1-Sulfo-2-naphthylamino, 4,8-Disulfo-2-naphthylamino, N-Ethyl-N-phenylamino, N-Methyl-N-phenylamino, Methoxy, Ethoxy, n- oder iso-Propoxy sowie Hydroxy.

Als nicht-reaktiver Rest hat T vorzugsweise die Bedeutung $C_1$-$C_4$-Alkoxy, $C_1$-$C_4$-Alkylthio, Hydroxy, Amino, N-$C_1$-$C_4$-Alkylamino, das im Alkylteil unsubstituiert oder durch Hydroxy, Sulfato oder Sulfo substituiert ist, Morpholino, Phenylamino oder N-$C_1$-$C_4$-Alkyl-N-phenylamino, worin das Phenyl jeweils unsubstituiert oder durch Sulfo, Carboxy, Acetylamino, Methyl oder Methoxy substituiert ist.

Besonders bevorzugte nicht-reaktive Reste T sind Amino, N-Methylamino, N-Ethylamino, β-Sulfoethylamino, Morpholino, Phenylamino, 2-, 3- oder 4-Sulfophenylamino oder N-$C_1$-$C_4$-Alkyl-N-phenylamino.

X bedeutet bevorzugt Halogen, z.B. Fluor, Chlor oder Brom und insbesondere bevorzugt Chlor oder Fluor.

$X_2$ bedeutet vorzugsweise Brom.

Bei alk und alk' handelt es sich unabhängig voneinander z.B. um einen Methylen-, Äthylen-, 1,3-Propylen-, 1,4-Butylen-, 1,5-Pentylen- oder 1,6-Hexylenrest oder deren verzweigte Isomere.

Bevorzugt stehen alk und alk' unabhängig voneinander je für einen $C_1$-$C_4$-Alkylenrest und insbesondere

bevorzugt für einen Ethylenrest oder Propylenrest.

arylen ist vorzugsweise ein unsubstituierter oder z.B. durch Sulfo, Methyl, Methoxy oder Carboxy substituierter 1,3- oder 1,4-Phenylenrest und besonders bevorzugt ein unsubstituierter 1,3- oder 1,4-Phenylenrest.

$R_2$ ist vorzugsweise Wasserstoff oder $C_1$-$C_4$-Alkyl, wie Methyl, Ethyl, Propyl, Isopropyl, Butyl, Isobutyl, sek.-Butyl oder tert.-Butyl. Besonders bevorzugt ist $R_2$ Wasserstoff.

$R_3$ bedeutet bevorzugt Wasserstoff.

Q steht vorzugsweise für -NH- oder -O- und insbesondere bevorzugt für -O-.

W bedeutet bevorzugt eine Gruppe der Formel -CONH- oder -NHCO-, insbesondere eine Gruppe der Formel -CONH-.

Bevorzugt als Reaktivreste der Formeln (3a) bis (3f) sind solche, worin W eine Gruppe der Formel -CONH- oder -NHCO-, $R_1$, $R_2$ und $R_3$ je Wasserstoff, Q der Rest -O- oder -NH- , alk und alk' unabhängig voneinander je Ethylen oder Propylen, arylen unsubstituiertes oder durch Methyl, Methoxy, Carboxy oder Sulfo substituiertes Phenylen, Y Vinyl oder β-Sulfatoethyl und $Y_1$ $-CHBr-CH_2Br$ oder $-CBr=CH_2$ bedeuten.

Weist der Rest K einen Reaktivrest auf, handelt es sich besonders bevorzugt um einen Rest der zuvor angegebenen Formel (2a); (2b), worin $R_1$ Wasserstoff und n die Zahl 2 oder 3 bedeuten; oder (2c), worin T $C_1$-$C_4$-Alkoxy, $C_1$-$C_4$-Alkylthio, Hydroxy, Amino, N-$C_1$-$C_4$-Alkylamino, das im Alkylteil unsubstituiert oder durch Hydroxy, Sulfato oder Sulfo substituiert ist, Morpholino, Phenylamino oder N-$C_1$-$C_4$-Alkyl-N-phenylamino, worin das Alkyl gegebenenfalls durch Hydroxy, Sulfo oder Sulfato und das Phenyl jeweils unsubstituiert oder durch Sulfo, Carboxy, Acetylamino, Methyl oder Methoxy substituiert ist, oder einen faserreaktiven Rest der Formel

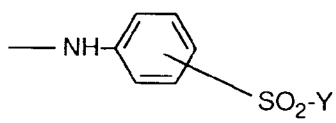

(3c')

oder

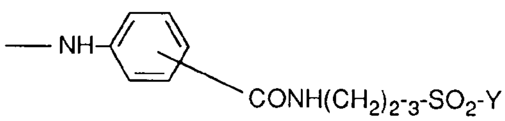

(3d')

bedeutet, wobei Y jeweils die zuvor angegebene Bedeutung hat.

Bedeutet s in Formel (1) die Zahl 1, steht K z.B. für einen Rest der Formel

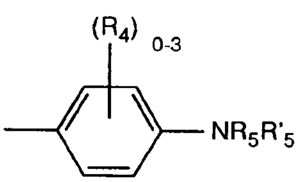 (4a)

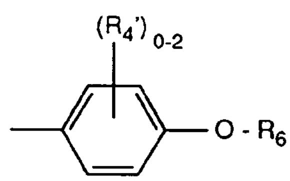 (4b)

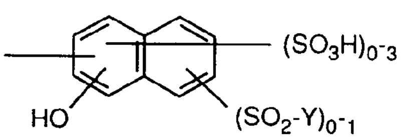 (4c)

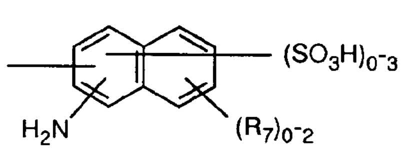 (4d)

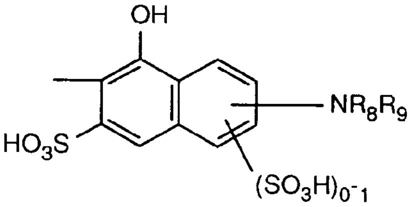 (4e)

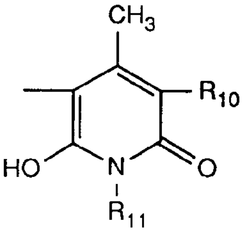 (4f)

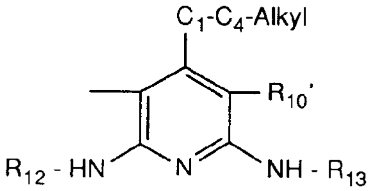 (4g)

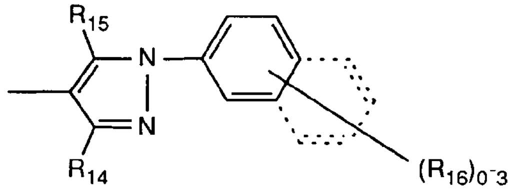 (4h)

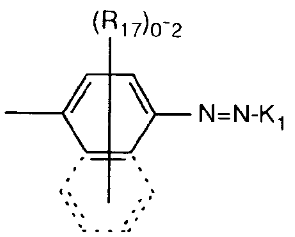 (4i)

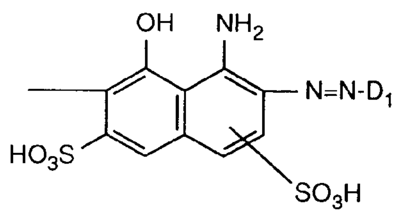 (4j)

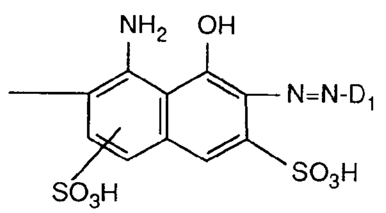 (4k),

oder

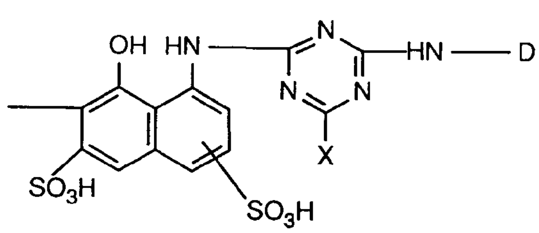 (4l)

worin

$(R_4)_{0-3}$ für 0 bis 3 gleiche oder verschiedene Reste $R_4$ z.B. aus der Gruppe $C_1$-$C_4$-Alkyl, $C_1$-$C_4$-Alkoxy, im Alkylteil durch Hydroxy, $C_1$-$C_4$-Alkoxy oder Sulfato substituiertes $C_1$-$C_4$-Alkoxy, Halogen, Carboxy, Hydroxy, Amino, N-Mono- oder N,N-Di-$C_1$-$C_2$-Alkylamino, $C_2$-$C_4$-Alkanoylamino, Ureido, Sulfo, $C_1$-$C_4$-Alkylsulfonyloxy, $C_1$-$C_4$-Alkylsulfonylamino oder einen Reaktivrest der zuvor angegebenen Formel (2c) steht;

$(R_4')_{0-2}$ für 0 bis 2 gleiche oder verschiedene Reste $R_4$, aus der Gruppe $C_1$-$C_4$-Alkyl, $C_1$-$C_4$-Alkoxy, Phenoxy, Halogen, Sulfo und Hydroxy steht;

$R_5$ Wasserstoff oder $C_1$-$C_4$-Alkyl ist;

$R_5$' unabhängig die Bedeutung von $R_5$ hat oder für $C_2$-$C_4$-Alkanoyl, $C_1$-$C_4$-Hydroxyalkyl, $C_1$-$C_4$-Sulfatoalkyl, $C_1$-$C_4$-Alkoxyalkyl oder gegebenenfalls durch Methyl, Methoxy, Chlor oder Sulfo substituiertes Phenyl steht;
$R_6$ Wasserstoff, $C_1$-$C_4$-Alkyl oder Phenyl bedeutet;
$(R_7)_{0-2}$ für 0 bis 2 gleiche oder verschiedene Substituenten aus der Gruppe Hydroxy und faserreaktiver Rest der zuvor angegebenen Formel (2a) steht;
$R_8$ Wasserstoff oder gegebenenfalls durch Hydroxy, $C_1$-$C_4$-Alkoxy, Sulfato, Sulfo, Halogen oder Cyano substituiertes $C_1$-$C_4$-Alkyl ist;
$R_9$ unabhängig die Bedeutung von $R_8$ hat oder $C_2$-$C_4$-Alkanoyl, Benzoyl oder einen faserreaktiven Rest der zuvor angegebenen Formel (2c) bedeutet;
$R_{10}$ und $R_{10}$' unabhängig voneinander je Carbamoyl, Sulfomethyl oder Cyano sind;
$R_{11}$ für Wasserstoff oder $C_1$-$C_4$-Alkyl, welches gegebenenfalls durch einen Rest der Formel (2c) substituiert ist, steht;
$R_{12}$ und $R_{13}$ unabhängig voneinander je für Wasserstoff oder gegebenenfalls durch Hydroxy, $C_1$-$C_4$-Alkoxy, Phenoxy, Sulfato, Sulfo, Carboxy, Amino oder N-Mono- oder N,N-Di-$C_1$-$C_4$-Alkylamino, welches seinerseits im Alkylteil durch Hydroxy, $C_1$-$C_4$-Alkoxy, Phenoxy, Sulfato, Sulfo, Carboxy oder einen faserreaktiven Rest der zuvor angegebenen Formel (2c) substituiert sein kann, substituiertes und/oder mit Ausnahme von Methyl gegebenenfalls durch -O- unterbrochenes $C_1$-$C_{12}$-Alkyl stehen;
$R_{14}$ Methyl oder Carboxy bedeutet;
$R_{15}$ Hydroxy oder Amino ist;
$(R_{16})_{0-3}$ für 0 bis 3 gleiche oder verschiedene Reste $R_{16}$ aus der Gruppe Sulfo, Halogen, Hydroxy, $C_1$-$C_4$-Alkoxy und $C_1$-$C_4$-Alkyl steht;
$(R_{17})_{0-2}$ für 0 bis 2 gleiche oder verschiedene Reste $R_{17}$ aus der Gruppe $C_1$-$C_4$-Alkyl, $C_1$-$C_4$-Alkoxy, im Alkylteil durch Hydroxy, $C_1$-$C_4$-Alkoxy oder Sulfato substituiertes $C_1$-$C_4$-Alkoxy, $C_2$-$C_4$-Alkanoylamino, Ureido, Halogen und Sulfo steht;
$K_1$ unabhängig einen Rest der zuvor angegebenen Formel (4a) - (4h) oder (4j) - (4l) bedeutet;
$D_1$ ein Phenyl- oder 1- oder 2-Naphthylrest ist, der 1 bis 3 gleiche oder verschiedene Reste aus der Gruppe Sulfo, $C_1$-$C_4$-Alkyl, $C_1$-$C_4$-Alkoxy, Halogen oder faserreaktiver Rest der Formel (2a) oder (2b) trägt;
D der Rest eines Monoazo- oder Disazochromophors ist;
und X und Y jeweils die zuvor angegebene Bedeutung haben.

Für die in den Formeln (4a) bis (4l) angegebenen Variablen gelten die folgenden Bevorzugungen:
$(R_4)_{0-3}$ steht bevorzugt für 0 bis 3 gleiche oder verschiedene Reste $R_4$ aus der Gruppe Methyl, Methoxy, im Alkylteil durch Hydroxy, Methoxy, Ethoxy oder Sulfato substituiertes Ethoxy, Chlor, Hydroxy, Amino, N-Mono- oder N,N-Di-$C_1$-$C_2$-Alkylamino, Acetylamino, Propionylamino, Sulfo, oder einen Reaktivrest der zuvor angegebenen Formel (2c), worin X Halogen, $R_1$ Wasserstoff und T gegebenenfalls durch nicht-reaktive Reste substituiertes Amino oder Morpholino bedeutet;
$(R_4')_{0-2}$ steht bevorzugt für 0 bis 2 gleiche oder verschiedene Reste $R_4$' aus der Gruppe Methyl, Methoxy, Phenoxy, Chlor, Sulfo und Hydroxy steht;
$R_5$ Wasserstoff, Methyl oder Ethyl ist;
$R_5$' unabhängig die Bedeutung von $R_5$ hat oder für Acetyl, Propionyl, 2-Hydroxyethyl oder 2-Sulfatoethyl;
$R_6$ bedeutet vorzugsweise Wasserstoff;
$R_8$ ist vorzugsweise Wasserstoff;
$R_9$ bedeutet vorzugsweise Acetyl, Propionyl, Benzoyl oder einen faserreaktiven Rest der zuvor angegebenen Formel (2c);
$R_{10}$ steht bevorzugt für Carbamoyl oder Sulfomethyl;
$R_{10}$' steht bevorzugt für Cyano oder Carbamoyl;
$R_{11}$ bedeutet bevorzugt Methyl oder Ethyl;
$R_{12}$ steht bevorzugt für Wasserstoff oder gegebenenfalls durch Hydroxy, Methoxy, Ethoxy, Sulfato oder Sulfo, substituiertes $C_1$-$C_6$-Alkyl;
$R_{13}$ hat unabhängig bevorzugt die Bedeutung von $R_{12}$ oder steht für einen Rest der zuvor angegebenen Formel (2c);
$R_{15}$ bedeutet bevorzugt Hydroxy;
$(R_{16})_{0-3}$ steht bevorzugt für 0 bis 3 gleiche oder verschiedene Reste $R_{16}$ aus der Gruppe Sulfo, Methyl, Methoxy, Hydroxy und Chlor;
$(R_{17})_{0-2}$ bedeutet bevorzugt 0 bis 2 gleiche oder verschiedene Reste $R_{17}$ aus der Gruppe Methyl, Methoxy, im Alkylteil durch Hydroxy oder Sulfato substituiertes $C_1$-$C_2$-Alkoxy, Acetylamino, Propionylamino und Sulfo;
$K_1$ steht bevorzugt für einen Rest der zuvor angegebenen Formel (4e);
$D_1$ ist bevorzugt ein Phenyl- oder 1- oder 2-Naphthylrest, der 1 bis 3 gleiche oder verschiedene Reste aus der

Gruppe Sulfo, Methyl, Methoxy, Chlor und faserreaktiver Rest der Formel (2a) trägt; und
D bedeutet bevorzugt den Rest eines Monoazo- oder Disazochromophors, der Diazokomponenten und Kupplungskomponenten aus der Benzol- oder Naphthalinreihe aufweist, die durch einen oder mehrere Substituenten aus der Gruppe Sulfo, Methyl, Methoxy, Chlor, Amino, Hydroxy, Acetylamino, Propionylamino, Benzoylarnino, Ureido, 2-Hydroxyethoxy, 2-Sulfatoethoxy und faserreaktiver Rest der Formel (2c) substituiert sind. Besonders bevorzugt steht D in Formel (41) für einen Rest der Formel

$$-D^* - N = N - K^* ( - N = N - D_1^*)_{0-1} \quad (5a)$$

oder

$$- K^* - N = N - (M - N = N - )_{0-1} D^* \quad (5b),$$

worin D* ein unsubstituierter oder durch Sulfo substituierter Phenylenrest ist, $D_1$* für unsubstituiertes oder durch Sulfo oder einen Rest der zuvor angegebenen Formel (2a) substituiertes Phenyl oder Naphthyl steht, K* den Rest einer Kupplungskomponente der Aminonaphtholsulfosäure-Reihe, z.B. den Rest von H-, K-, I- oder γ-Säure bedeutet, der gegebenenfalls an der Aminogruppe durch Acetyl, Propionyl, Benzoyl oder einen faserreaktiven Rest der zuvor angegebenen Formel (2c) substituiert ist, und M gegebenenfalls durch Sulfo, Methyl, Methoxy, Acetylamino, Ureido, 2-Hydroxyethoxy oder 2-Sulfatoethoxy substituiertes Phenylen bedeutet.

Bedeutet s in Formel (1) die Zahl 1, steht K bevorzugt für einen Rest der Formel

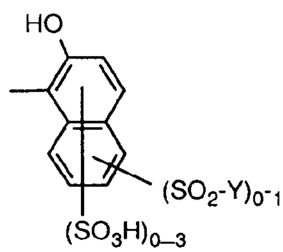 (4c'),

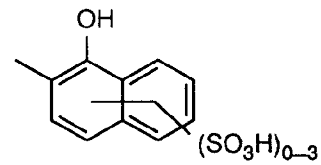 (4c''),

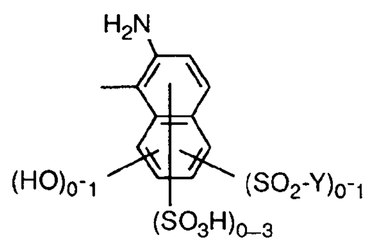 (4d'),

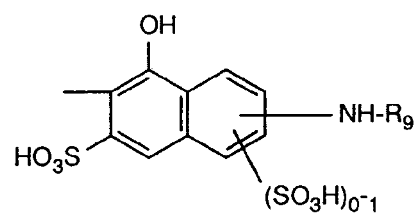 , (4e')

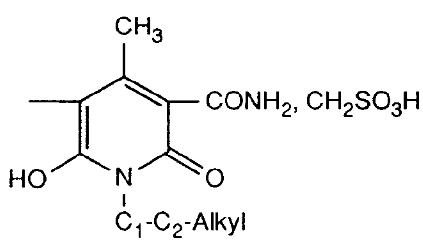 (4f'),

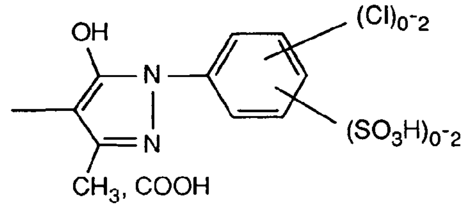 (4h'),

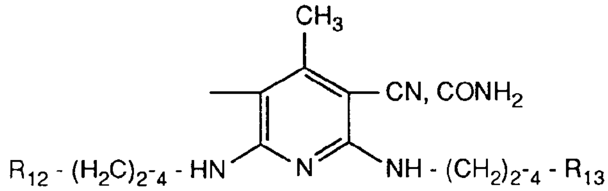 (4g'),

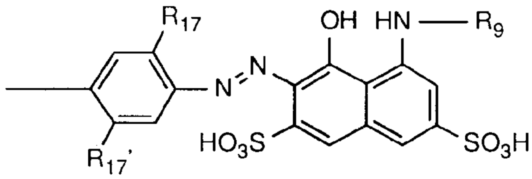

(4i'),

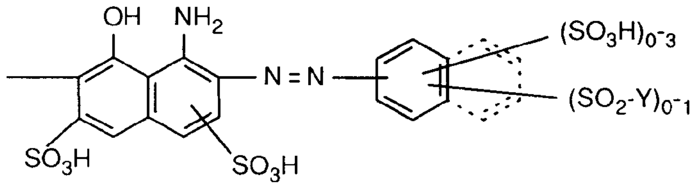

(4j')

oder

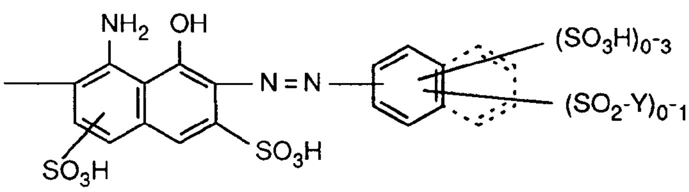

(4k'),

worin $R_9$ Acetyl, Benzoyl oder einen Rest der zuvor angegebenen Formel (2c) bedeutet, $R_{12}$ Wasserstoff, Hydroxy oder Sulfato ist, $R_{13}$ unabhängig die Bedeutung von $R_{12}$ hat oder für einen Rest der zuvor angegebenen Formel (2c) steht, $R_{17}$ und $R_{17}'$ unabhängig voneinander je für Wasserstoff, Methyl, Methoxy, 2-Hydroxyethoxy, 2-Sulfatoethoxy, Acetylamino, Propionylamino, Ureido oder Sulfo stehen, und für Y die zuvor angegebenen Bedeutungen und Bevorzugungen gelten.

Bedeutet s die Zahl 2, so ist K z.B. der Rest einer doppelt ankuppelbaren Kupplungskomponente der Aminonaphtholsulfonsäure-Reihe oder ein Rest der Formel

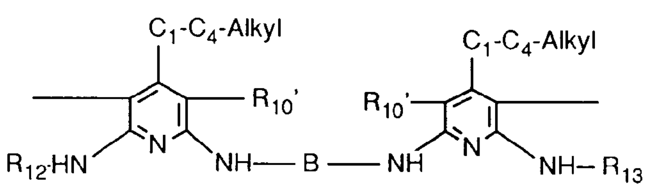

(6)

oder

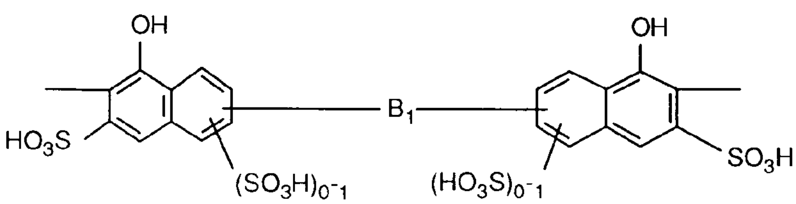

(7),

worin $B_1$ ein Brückenglied der Formel

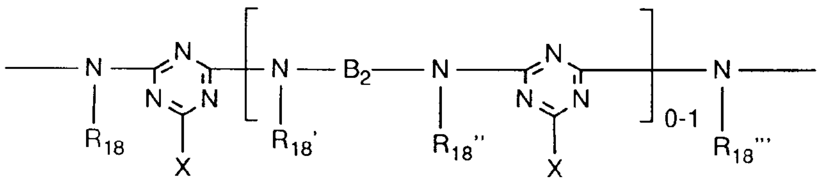 (8),

bedeutet, $R_{18}$, $R_{18}$', $R_{18}$", und $R_{18}$"' unabhängig voneinander je Wasserstoff oder unsubstituiertes oder z.B. durch Halogen, Hydroxy, Cyano, $C_1$-$C_4$-Alkoxy, Carboxyl, Sulfo oder Sulfato substituiertes $C_1$-$C_4$-Alkyl sind, $B_2$ ein aliphatisches, cycloaliphatisches oder aromatisches Brückenglied bedeutet und für $R_{10}$', $R_{12}$, $R_{13}$ und X jeweils die zuvor angegebenen Bedeutungen und Bevorzugungen gelten.

Geeignete doppelt ankuppelbare Kupplungskomponenten der Aminonaphtholsulfonsäure--Reihe K sind z.B. 1-Amino-8-hydroxynaphthalin-3,6-disulfonsäure (H-Säure) oder 1-Amino-8-hydroxynaphthalin-4,6-disulfonsäure (K-Säure).

Bei dem Alkylenrest B kann es sich z.B. um geradkettiges oder verzweigtes und gegebenenfalls durch -O- unterbrochenes $C_2$-$C_{12}$-Alkylen handeln. Bevorzugt steht B für geradkettiges oder verzweigtes $C_2$-$C_6$-Alkylen, welches gegebenenfalls durch Hydroxy, Sulfo oder Sulfato substituiert ist. Beispiele für besonders bevorzugte Alkylenreste B sind 1,2-Ethylen, 1,2-Propylen, 1,3-Propylen, 2-Hydroxy-1,3-Propylen, 2-Methyl-1,5-pentylen und 1,6-Hexylen.

$R_{18}$, $R_{18}$', $R_{18}$" und $R_{18}$"' in Formel (8) bedeuten unabhängig voneinander je bevorzugt Wasserstoff, Methyl oder Ethyl und besonders bevorzugt je Wasserstoff.

Bedeutet $B_2$ ein aliphatisches Brückenglied, so gelten hierbei die zuvor für B genannten Bedeutungen und Bevorzugungen.

Steht $B_2$ für ein cycloaliphatisches Brückenglied, kommt hierbei z.B. Cyclohexylen oder der Rest der Formel

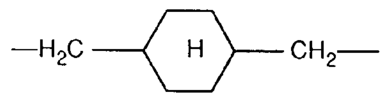 (9)

in Frage, oder -$NR_{18}$' und -$NR_{18}$" bilden zusammen mit $B_2$ einen Piperazinring.

Beispiele für aromatische Brückenglieder $B_2$ sind unsubstituiertes oder z.B. durch Sulfo, Methyl, Methoxy oder Chlor substituiertes 1,2-, 1,3- oder 1,4-Phenylen, gegebenenfalls durch Sulfo substituiertes Naphthylen, gegebenenfalls durch Sulfo substituiertes Benzylen oder ein Rest der Formel

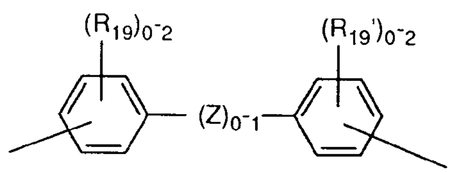 (10),

worin Z z.B. die Bedeutung -CO-, -NHCO-, -NHCONH-, -$(CH_2)_{1-4}$-, -NH-, -CH=CH-, -O-, -$SO_2$- oder -N=N- hat und $(R_{19})_{0-2}$ und $(R_{19}')_{0-2}$ unabhängig voneinander jeweils für 0 bis 2 gleiche oder verschiedene Reste aus der Gruppe Sulfo, Methyl, Methoxy und Chlor stehen.

Bevorzugt als aromatisches Brückenglied $B_2$ sind unsubstituiertes oder durch Sulfo, Methyl oder Methoxy substituiertes 1,3- oder 1,4-Phenylen, durch 1 oder 2 Sulfogruppen substituiertes Naphthylen oder ein Rest der Formel

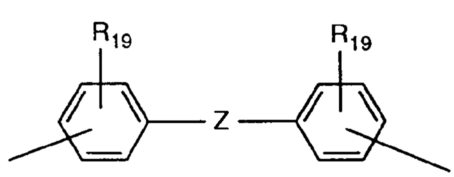 (10a)

oder

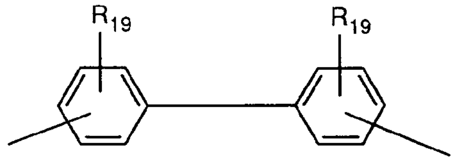
(10b),

worin Z -NHCONH-, -O-, -NH-, -CH=CH- oder $-CH_2-$ und $R_{19}$ Wasserstoff oder Sulfo bedeuten.

Beispiele für besonders bevorzugte aromatische Brückenglieder $B_2$ sind 1,3-Phenylen, 1,4-Phenylen, 4-Methylphenylen-1,3,4-Sulfophenylen-1,3,3-Sulfophenylen-1,4, 3,6-Disulfophenylen-1,4, 4,6-Disulfophenylen- 1,3, 3,7-Disulfonaphthylen-1,5, 4,8-Disulfonaphthylen-2,6, 2,2'-Disulfodiphenylen-4,4', 4,4'-Phenylenharnstoff--2,2'-disulfonsäure und 2,2'-Disulfostilbenylen-4,4'.

Bedeutet s die Zahl 2, so ist K vorzugsweise ein Rest der Formel

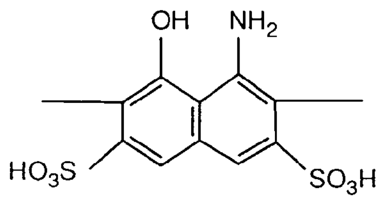
(11a) (Rest von H-Säure),

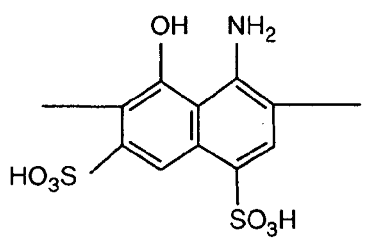
(11b) (Rest von K-Säure),

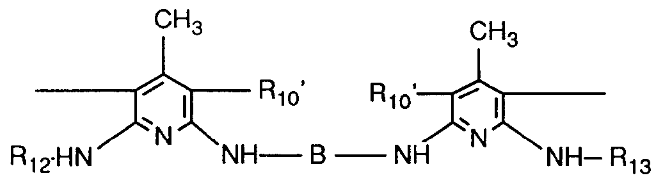
(6a),

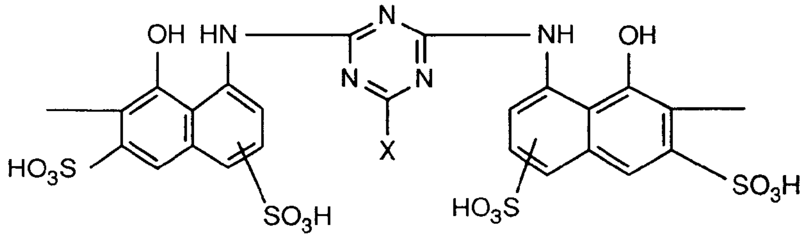
(7a),

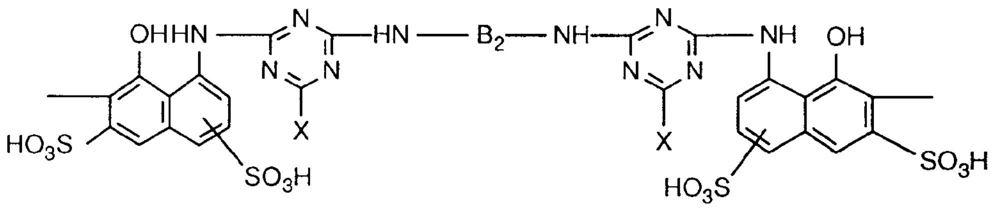

(7b),

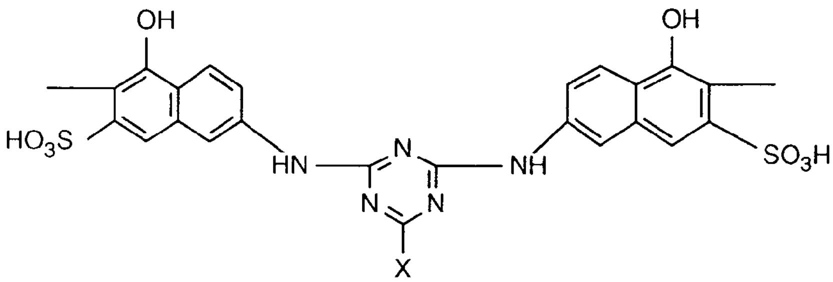

(7c)

oder

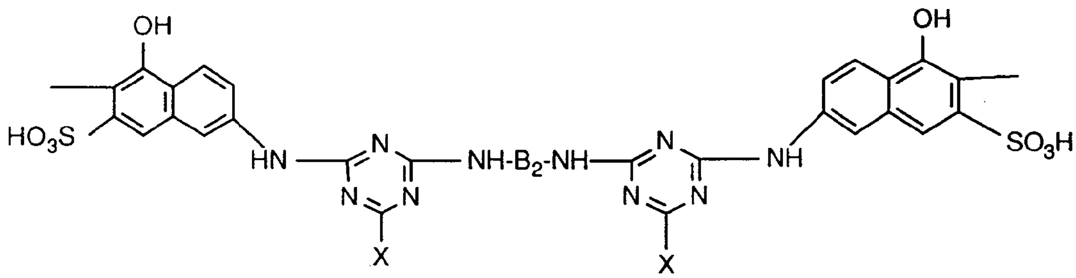

(7d),

worin $R_{10}$' Cyano oder Carbamoyl ist, $R_{12}$ und $R_{13}$ unabhängig voneinander jeweils für Wasserstoff oder gegebenenfalls durch Hydroxy, Methoxy, Ethoxy, Sulfato oder Sulfo, substituiertes $C_1$-$C_6$-Alkyl stehen, B geradkettiges oder verzweigtes $C_2$-$C_6$-Alkylen, welches gegebenenfalls durch Hydroxy, Sulfo oder Sulfato substituiert sein kann, bedeutet, $B_2$ unabhängig die Bedeutung von B hat oder für unsubstituiertes oder durch Sulfo, Methyl oder Methoxy substituiertes 1,3- oder 1,4-Phenylen, durch 1 oder 2 Sulfogruppen substituiertes Naphthylen oder für einen Rest der zuvor angegebenen Formel (10a) oder (10b) steht, und für X die zuvor genannten Bedeutungen und Bevorzugungen gelten.

Bedeutet s die Zahl 2, so ist K besonders bevorzugt ein Rest der zuvor angegebenen Formel (11a), (7a), (7b), (7c) oder (7d), worin $B_2$ geradkettiges oder verzweigtes $C_2$-$C_6$-Alkylen, welches gegebenenfalls durch Hydroxy, Sulfo oder Sulfato substituiert sein kann, bedeutet und X für Chlor oder Fluor steht.

Die Verbindungen der Formel (1) können erhalten werden, z.B. indem man in etwa s Moläquivalente der Verbindung der Formel

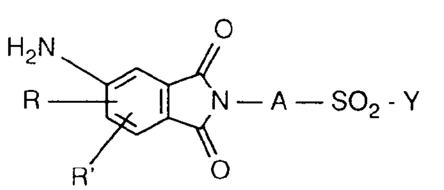

(12)

in üblicher Weise, z.B. mit Nitriten wie Natriumnitrit im sauren, z.B. salzsauren, Medium, diazotiert und auf in etwa 1 Moläquivalent einer Kupplungskomponente der Formel

$$K - (H)_s \qquad (13),$$

kuppelt, worin für A, K, R, R', Y und s jeweils die zuvor genannten Bedeutungen und Bevorzugungen gelten.

Die Kupplung der diazotierten Verbindung der Formel (12) mit der Verbindung der Formel (13) findet vorteilhaft bei einem neutralen bis leicht sauren pH-Wert, z.B. bei pH 3 bis 7 und bevorzugt 5 bis 6,5, und Temperaturen von -5 bis 30°C und bevorzugt 0 bis 25°C statt.

Die Verbindungen der Formel (12) können erhalten werden z.B., indem man

a) Phthalsäureanhydrid mit einer Verbindung der Formel

$$H_2N - A - S - CH_2\text{-}CH_2\text{-}U_1 \qquad (14)$$

zur Verbindung der Formel

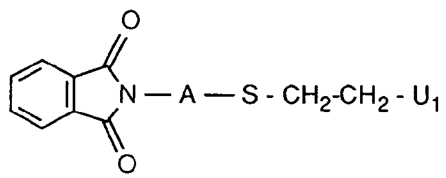
(15),

umsetzt, worin für A jeweils die zuvor angegebenen Bedeutungen und Bevorzugungen gelten und $U_1$ unabhängig die zuvor für U angegebene Bedeutung hat oder für Hydroxy steht,

b) die Thioetherverbindung der Formel (15) zur entsprechenden Sulfonylverbindung der Formel

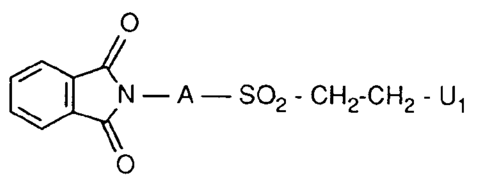
(15a)

oxidiert,

c) die erhaltene Verbindung der Formel (15a) selektiv nitriert zur Verbindung der Formel

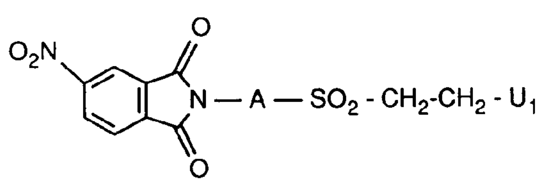
(15b),

d) die erhaltene Verbindung der Formel (15b) zur Aminoverbindung der Formel

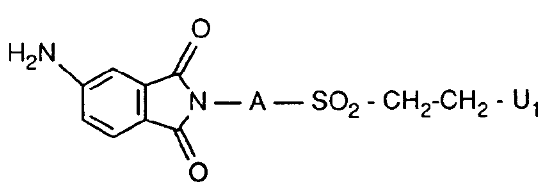
(15c)

reduziert und gegebenenfalls während oder nach der Synthese der Verbindung der Formel (15a), (15b) oder (15c) einen der zuvor genannten Reste R und/oder R' in das Molekül einführt oder den Rest $U_1$ in einen beliebigen Rest U umwandelt.

Die Kondensation des Phthalsäureanhydrids mit dem Amin der Formel (14) im Schritt a) erfolgt z.B. in wässrigem oder wässrig-organischem Medium bei einer Temperatur von ca. 40 bis 200°C und vorzugsweise 50 bis 150°C und Normaldruck unter Abdestillieren des entstehenden Wassers.

Die Oxidation der Thioetherverbindung der Formel (15) zur Sulfonylverbindung der Formel (15a) im Schritt b) kann nach verschiedenen Methoden erfolgen, z.B. mit Wasserstoffperoxid mit oder ohne Zusatz von Wolfram- oder Vanadinverbindungen als Katalysator, ferner mit Peressigsäure, Kaliumpermanganat oder Chromsäure, oder mit Chlor/Salzsäure je in wässrigem, wässrig-organischem oder organischem Medium.

Die Nitrierung der Verbindung der Formel (15a) zur Verbindung der Formel (15b) im Schritt c) findet vorteilhaft in einem Gemisch von konz. Schwefelsäure und konz. Salpetersäure bei Raumtemperatur oder darüber, z.B. bei einer Temperatur von10 bis 100°C und vorzugsweise 30 bis 50°C, statt.

Die Reduktion der Nitroverbindung der Formel (15b) zur Aminoverbindung der Formel (15c) im Schritt d) erfolgt z.B. durch katalytische Hydrierung mit Pd/Kohlenstoff in wässrigem, wässrig-organischem oder organischem Medium bei Raumtemperatur bis etwa 60°C; enthält das zur Hydrierung verwendete Medium organische Lösungsmittel, so kommen hierbei in erster Linie Alkohole wie Ethanol sowie Essigester oder Tetrahydrofuran in Frage. Das Hydriergemisch kann auch einen Puffer enthalten, z.B. einen Essigsäure/Acetatpuffer. Die Reduktion kann auch mit Fe/Salzsäure oder Fe/Essigsäure in wässriger Lösung durchgeführt werden.

Die Verbindungen der Formel (15c), worin $U_1$ Hydroxy bedeutet, können durch Behandlung mit Sulfatierungsmitteln, Phosphorylierungsmitteln, Halogenierungsmitteln oder Alkyl- oder Arylcarbonsäurehalogeniden wie Benzoylchlorid oder Acetylchlorid in die entsprechenden Verbindungen der Formel (12), worin Y einen Rest $-CH_2-CH_2-U$ ist, überführt werden.

Geeignete Sulfatierungsmittel sind beispielsweise konz. Schwefelsäure sowie Chlorsulfonsäure und Amidosulfonsäure oder andere $SO_3$ abgebenden Verbindungen. Die Sulfatierung der Hydroxygruppe $U_1$ zur Sulfatogruppe U kann auch im Verlauf der Synthese der Verbindungen der Formel (15c), z.B. im Nitrierungsschritt c), erfolgen. Als Halogenierungsmittel können beispielsweise Thionylchlorid oder Thionylbromid verwendet werden. Geeignete Phosphorylierungsmittel sind z.B. konz. Phosphorsäure, Pyro-, Meta- oder Polyphosphorsäure, Phosphorsäurealkylester, Phosphoroxytrichlorid oder Gemische von Phosphorsäure und Phosphor-(V)-oxid.

Wird in die Verbindung der Formel (15c) ein Rest R eingeführt, so kommt hier z.B. die Sulfonierung oder Halogenierung, d.h. die Einführung einer Sulfo- oder Halogengruppe, in Frage, die in an sich bekannter Weise, z.B. mit konz. Schwefelsäure, Oleum oder Chlorsulfonsäure bzw. mit Chlor oder Brom in Gegenwart von Lewissäuren, erfolgen kann. Die Verbindungen der Formel (12) mit R = Sulfo oder Halogen können wiederum als Ausgangsprodukte für die Synthese der entsprechenden Verbindungen mit R = Hydroxy, $C_1$-$C_4$-Alkoxy oder Cyano dienen, die nach üblichen in den Lehrbüchern der organischen Chemie beschriebenen Methoden erfolgen kann.

Die Kupplungskomponenten der Formel (13) sind bekannt oder können in an sich bekannter Weise erhalten werden.

Die neuen Verbindungen der Formel (1) sind faserreaktiv, d.h. sie vermögen mit den Hydroxylgruppen der Cellulose oder mit den reaktiven Zentren von natürlichen und synthetischen Polyamiden unter Bildung kovalenter chemischer Bindungen zu reagieren.

Ein weiterer Gegenstand der vorliegenden Erfindung betrifft Verbindungsgemische enthaltend je eine Verbindung der Formel

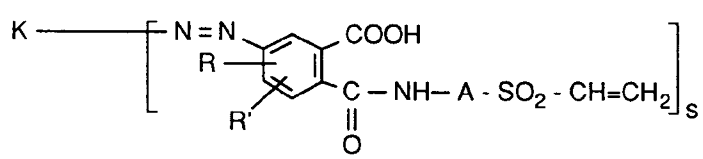
(16a)

und

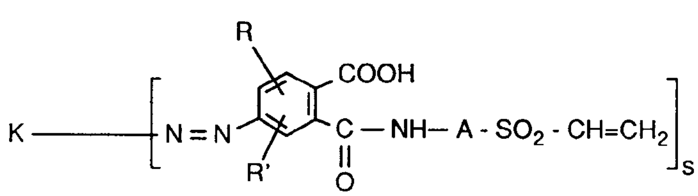
(16b),

worin für A, K, R, R' und s jeweils die zuvor angegebenen Bedeutungen und Bevorzugungen gelten; die genannten Verbindungsgemische stellen ebenfalls wertvolle neue faserreaktive Farbstoffe für Cellulose- und Polyamidfasermaterialien dar. Die Verbindungsgemische der Formel (16a) und (16b) können z.B. durch einfache Behandlung der erfindungsgemässen Verbindungen der Formel (1) in einem wässrigen alkalischen Medium erhalten werden; alternativ kann man zunächst eine Verbindung der Formel (12) im alkalischen Medium behandeln und das dabei erhaltene Gemisch der Verbindungen der Formel

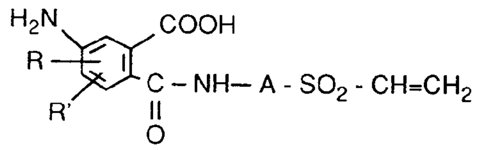
(12a)

und

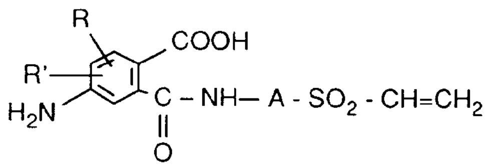
(12b),

worin für A, R und R' jeweils die zuvor angegebenen Bedeutungen und Bevorzugungen gelten, anschliessend diazotieren und auf eine Kupplungskomponente der zuvor angegebenen Formel (13) kuppeln. Das alkalische Medium weist jeweils z.B. einen pH-Wert von 8-14 und vorzugsweise 9-12 auf und kann mittels üblicher Basen, z.B. Natriumhydroxid, hergestellt werden. Beim Behandeln der Verbindungen der Formel (1) bzw. (12) in einem solchen alkalischen Medium tritt eine Ringöffnung ein, wobei die Komponenten der Formel (16a) und (16b) bzw. (12a) und (12b) im allgemeinen in einem stöchiometrischen Verhältnis, d.h. in etwa im Verhältnis 1:1, anfallen.

Die Reaktivfarbstoffe der Formel (1) sowie (16a) und (16b) eignen sich zum Färben und Bedrucken der verschiedensten Materialien, insbesondere hydroxylgruppenhaltigen oder stickstoffhaltigen Fasermaterialien. Beispiele sind Seide, Leder, Wolle, Polyamidfasern und Polyurethane sowie insbesondere cellulosehaltige Fasermaterialien aller Art. Solche Fasermaterialien sind beispielsweise die natürliche Cellulosefaser, wie Baumwolle, Leinen und Hanf, sowie Zellstoff und regenerierte Cellulose. Die Reaktivfarbstoffe der Formel (1) sowie (16a) und (16b) sind auch zum Färben oder Bedrucken von hydroxylgruppenhaltigen Fasern geeignet, die in Mischgeweben enthalten sind, z.B. von Gemischen aus Baumwolle mit Polyesterfasern oder Polyamidfasern.

Die erfindungsgemässen Farbstoffe lassen sich auf verschiedene Weise auf das Fasermaterial applizieren und auf der Faser fixieren, insbesondere in Form von wässrigen Farbstofflösungen und -druckpasten. Sie eignen sich sowohl für das Ausziehverfahren als auch zum Färben nach dem Foulardverfahren, können bei niedrigen Färbetemperaturen eingesetzt werden und erfordern bei Pad-Steam-Verfahren nur kurze Dämpfzeiten. Die Fixiergrade sind hoch, und die nicht fixierten Anteile können leicht ausgewaschen werden, wobei die Differenz zwischen Ausziehgrad und Fixiergrad bemerkenswert klein, d.h. der Seifverlust sehr gering ist. Die Reaktivfarbstoffe der Formel (1) sowie (16a) und (16b) eignen sich auch zum Druck, vor allem auf Baumwolle, ebenso aber auch zum Bedrucken von stickstoffhaltigen Fasern, z.B. von Wolle, Seide oder Wolle enthaltenden Mischgeweben.

Die mit den erfindungsgemässen Farbstoffen hergestellten Färbungen und Drucke auf Cellulosefasermaterialien besitzen eine hohe Farbstärke und eine hohe Faser-Farbstoff-Bindungsstabilität, sowohl in saurem als auch in alkalischem Bereich, weiterhin eine gute Lichtechtheit und sehr gute Nassechtheitseigenschaften, wie Wasch-, Wasser-, Seewasser-, Überfärbe- und Schweissechtheiten, sowie eine gute Plissierechtheit, Bügelechtheit, Reibechtheit und insbesondere Chlorechtheit.

Die nachfolgenden Beispiele dienen der Erläuterung der Erfindung. Die Temperaturen sind in Celsiusgraden angegeben, Teile sind Gewichtsteile, und Prozentangaben beziehen sich auf Gew.-%, sofern nicht anders vermerkt. Gewichtsteile stehen zu Volumenteilen im Verhältnis von Kilogramm zu Liter.

Herstellung der Verbindungen der Formel (12), (12a) und (12b)

Beispiel 1:

Zu einer Lösung aus 121 Teilen 2-Amino-2'-hydroxy-diethylsulfid in 121 Teilen Wasser werden 148 Teile Phthalsäureanhydrid eingetragen. Die Temperatur lässt man dabei auf 50°C ansteigen. Nach dem Eintragen erwärmt man die entstandene Suspension auf Rückfluss und destilliert bei Normaldruck das entstehende Wasser ab. Anschliessend erhöht man die Temperatur auf 150°C und lässt eine weitere Stunde bei dieser Temperatur rühren. Man erhält eine Schmelze von N-(2-(2-Hydroxy-ethylthio)-ethyl)-phthalimid der Formel

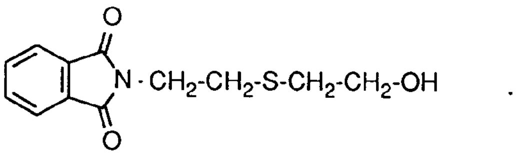

Man kühlt die entstandene Schmelze auf 80°C ab und gibt dann 8 Teile Natriumacetat und 0,5 Teile Wolfram(VI)-oxid dazu. Bei einer Temperatur von 75-80°C werden nun innert 2 Stunden 214 Teile Wasserstoffperoxid 35% zugetropft und weitere 2 Stunden bei 80°C und schwachem Peroxidüberschuss nachgerührt, bis mittels HPLC kein Sulfoxid mehr nachweisbar ist. Zur Ausfällung der entstandenen Emulsion giesst man die heisse Reaktionsmasse auf 500 Teile kaltes Wasser, filtriert und wäscht mit Wasser peroxidfrei. Nach dem Trocknen im Vakuum bei 60°C erhält man 253 Teile N-(2-(2-Hydroxy-ethylsulfonyl)-ethyl)-phthalimid der Formel

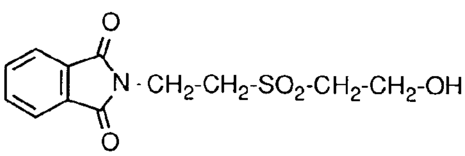

mit einem Schmelzpunkt von 120-122°C und einer Reinheit (HPLC) von 99%.
$^{1}$H-NMR-Analyse (gemessen in DMSO-$d_6$ mit TMS als Standard):
3,30 ppm (2H;t), 3,50 ppm (2H;t), 3,79 ppm (2H;q), 4,05 ppm (2H;t), 5,17 ppm (1H;t), 7,8 - 7,9 ppm (4H).

Beispiel 2:

Zu 735 Teilen Schwefelsäure 100% werden unter Rühren bei 20-30°C 253 Teile N-(2-(2-Hydroxyethylsulfonyl)ethyl)phthalimid eingetragen. Man lässt eine Stunde bei Raumtemperatur nachrühren bis eine vollständige Lösung vorliegt. Nun werden bei einer Temperatur von 10-15°C 113 Teile Mischsäure 50% innert 3 Stunden zugetropft. Danach erwärmt man auf 40°C und lässt weitere 12 Stunden bei dieser Temperatur nachrühren. Zur Isolierung trägt man das Nitriergemisch bei max. 5°C auf 2500 Teile Eis aus. Die klare Lösung wird mit 250 Teilen Kaliumsulfat versetzt und eine Stunde bei 10°C verrührt. Das ausgefallene Produkt wird abfiltriert und mit Kaliumsulfatlösung 10% gewaschen. Man erhält 1195 Teile feuchtes 4-Nitro-N-(2-(2-sulfato-ethylsulfonyl)-ethyl)-phthalimid der Formel

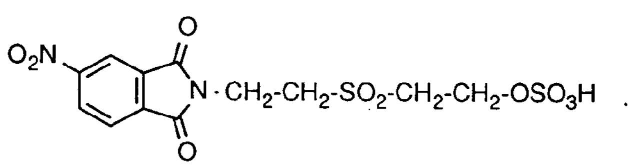

$^{1}$H-NMR-Analyse (gemessen in DMSO-$d_6$ mit TMS als Standard): 3,45 ppm (2H, t); 3,50 ppm (2H, t); 4,05 ppm (2H, t); 4,10 ppm (2H, t); 8,16 ppm (1H, d); 8,52 ppm (1H, s); 8,63 ppm (1H, d).

Beispiel 3:

1195 Teile feuchtes 4-Nitro-N-(2-(2-sulfato-ethylsulfonyl)-ethyl)-phthalimid werden in 2500 Teilen Wasser, 10 Teilen Natriumacetat und 8 Teilen Essigsäure gelöst. Diese Lösung wird unter Zugabe von 30 Teilen Palladium-Kohle 5% unter Normaldruck bei 50°C mit Wasserstoff bis zum Stillstand hydriert. Nach der Katalysatorfiltration wird die entstandene Lösung mit 700 Teilen Kaliumchlorid versetzt und das ausgefallene Produkt abgenutscht. Nach dem Trocknen im Vacuum bei 50°C erhält man 150 Teile 78%iges (Nitrittiter) 4-Amino-N-(2-(2-sulfato-ethylsulfonyl)-ethyl)-phthalimid der Formel

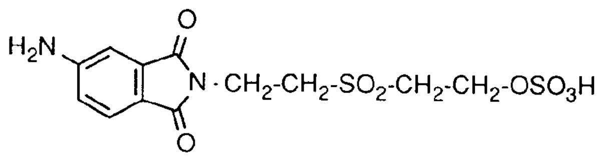

mit einer Reinheit von 96% (HPLC).
$^1$H-NMR-Analyse (gemessen in DMSO-$d_6$ mit TMS als Standard):
3,42 ppm (2H;t), 3,48 ppm (2H;t), 3,94 ppm (2H;t), 4,07 ppm (2H;t), 6,45 ppm (2H;s), 6,80 ppm (1H;q) $^3J$= 7 Hz und $^4J$= 2 Hz, 6,93 ppm (1H;d) $^4J$= 2 Hz, 7,48 ppm (1H,d) $^3J$= 7 Hz.

Beispiele 4-6a:

Analog wie in den Beispielen 1-3 beschrieben lassen sich die folgenden Verbindungen herstellen:

4

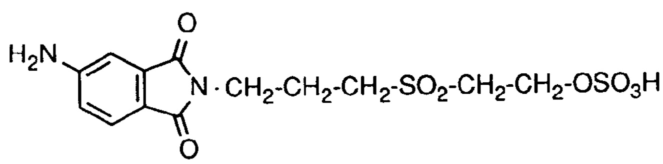

5

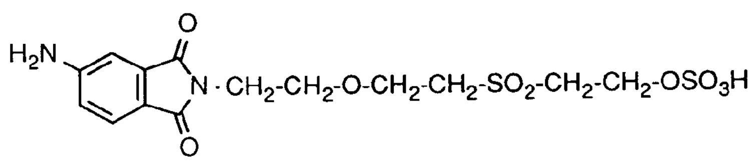

6

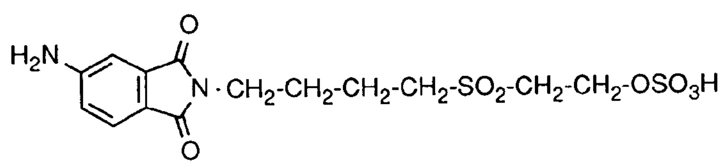

6a

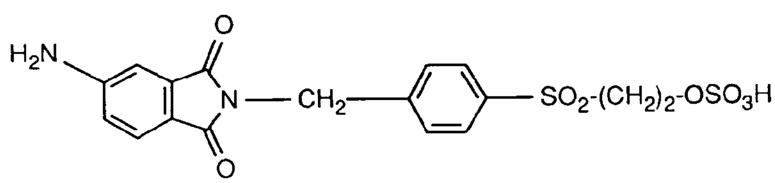

Beispiele 7- 10a:

Behandelt man die gemäss den Beispielen 3 bis 6a erhaltenen Verbindungen ca. 1 Stunde lang in einer wässrigen Lösung, deren pH-Wert durch Zugabe von Natriumhydroxidlösung auf ca. 10 eingestellt ist, und erniedrigt danach den pH-Wert mit Essigsäure auf 6, so erhält man ein in etwa stöchiometrisches Gemisch von zwei Verbindungen der in der Tabelle angegebenen Formel, worin sich die Aminogruppe einmal in 4-Stellung und einmal in 5-Stellung befindet.

7 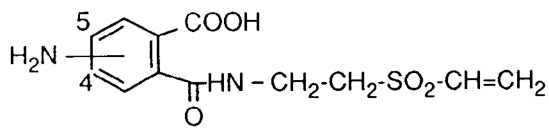

8 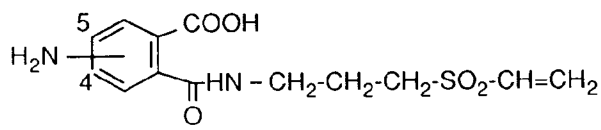

9 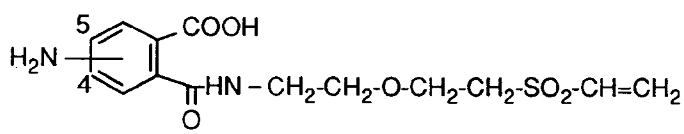

10 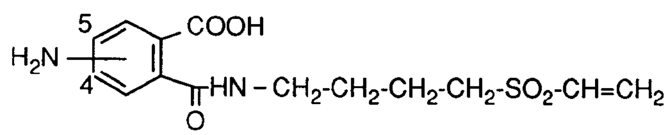

10a 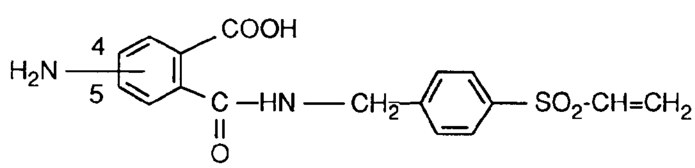

Beispiel 11:

Zu 215 Teilen 100%iger Schwefelsäure werden bei 20 bis 30°C 32 Teile 65%iges Oleum zugetropft. Danach trägt man 37,8 Teile der Verbindung gemäss Beispiel 3 innerhalb von 30 Minuten ein und lässt dabei die Temperatur auf 60°C ansteigen. Man erwärmt die Sulfiermasse anschliessend ca. 3 Stunden auf 130°C, lässt danach auf Raumtemperatur abkühlen und trägt die abgekühlte Reaktionsmasse auf 500 Teile Eis aus. Man fällt die überschüssige Schwefelsäure mit Calciumcarbonat aus und stellt den pH-Wert des nach dem Abfiltrieren des $CaSO_4$ resultierenden Filtrats mit Natriumcarbonat auf einen Wert von 4 ein. Durch Eindampfen oder Gefriertrocknen erhält man die Verbindung der Formel

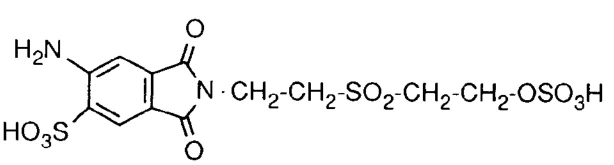

$^1$H-NMR-Analyse (gemessen in DMSO-$d_6$ mit TMS als Standard):
3,45 ppm (2H;t), 3,52 ppm (2H;t), 4,03 ppm (2H;t), 4,09 ppm (2H;t), 7,03 ppm (1H;s), 7,80 ppm (1H;s).

Beispiel 12:

Behandelt man die gemäss Beispiel 11 erhaltene Verbindung in einem alkalischen Medium, erhält man ein in etwa stöchiometrisches Gemisch von zwei Verbindungen der Formel

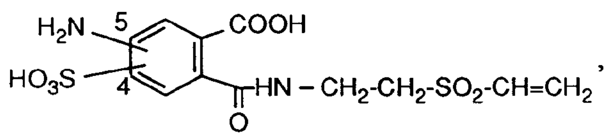

worin die eine Verbindung die Aminogruppe in 4-Stellung und die Sulfogruppe in 5-Stellung und die andere Verbindung die Aminogruppe in 5-Stellung und die Sulfogruppe in 4-Stellung aufweist.

Herstellung der Farbstoffe der Formel (1) und (16a) und (16b)

Beispiel 13:

37,8 Teile der Verbindung gemäss Beispiel 3 in 250 Teilen Eiswassersuspension werden mit 25 Volumenteilen 32%iger wässriger Salzsäure angesäuert und mit 25 Volumenteilen 4n Natriumnitritlösung diazotiert. Man rührt eine Stunde bei einer Temperatur von 5°C nach und zerstört dann die überschüssige salpetrige Säure mit Amidosulfonsäure.

Diese Diazoniumsuspension lässt man bei 0 - 5°C und einem pH von 4,5 - 5,5 langsam in eine Suspension bestehend aus bei pH 6 in 200 Teilen Wasser angeschlämmte 1-Benzoylamino-8-hydroxynaphthalin-3,6-disulfonsäure einlaufen. Der pH wird dabei unter Zugabe von Natriumcarbonatlösung gehalten. Nach der Zugabe rührt man weitere 2 Stunden bis zur vollständigen Kupplung bei pH 5,5 nach und lässt dabei die Temperatur auf Raumtemperatur ansteigen. Dann wird die Farbstofflösung umkehrosmotisiert und gefriergetrocknet. Man erhält einen Farbstoff, der in Form der freien Säure der Verbindung der Formel

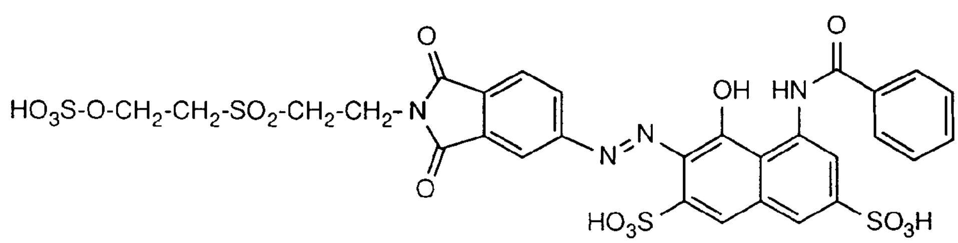

entspricht und Baumwolle und Wolle in brillanten roten Tönen mit guten Allgemeinechtheiten färbt.

Beispiel 14:

Verfährt man wie im Beispiel 13 angegeben und stellt den pH-Wert des Reaktionsgemisches vor der Umkehrosmose mit 4n Natronlauge auf pH 10, lässt eine Stunde bei diesem pH verrühren und stellt danach den pH mit etwas Essigsäure wieder auf pH 6, so erhält man einen Farbstoff, welcher in der Form der freien Säure dem Isomerengemisch im Verhältnis 1:1 der Formel

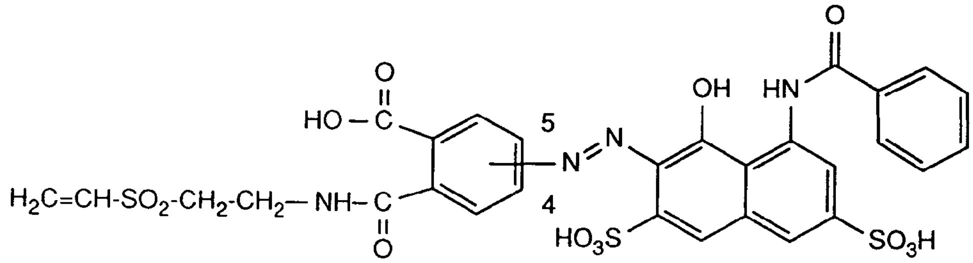

entspricht. Durch Umkehrosmose und anschliessendes Gefriertrocknen erhält man einen Farbstoff, der auf Baumwolle oder Wolle ausgefärbt die gleichen Eigenschaften wie der in Beispiel 13 angegebene Farbstoff besitzt.

Beispiel 15:

37,8 Teile 4-Amino-N-(2-(2-sulfato-ethylsulfonyl)-ethyl)-phthalimid gemäss Beispiel 3 in 250 Teilen Eiswassersuspension werden mit 25 Volumenteilen 32%iger wässriger Salzsäure angesäuert und mit 25 Volumenteilen 4n Natriumnitritlösung diazotiert. Man rührt eine Stunde bei einer Temperatur von 5°C nach und zerstört dann die überschüssige salpetrige Säure mit Amidosulfonsäure. Zu dieser Diazosuspension lässt man innerhalb von 30 Minuten eine mit 10%iger Natriumcarbonatlösung hergestellte neutrale Lösung aus 16 Teilen 1-Amino-8-hydroxynaphthalin-3,6-disulfosäure in 100 Teilen Wasser zulaufen. Man lässt 3 Stunden bei 0 - 5°C verrühren, stellt danach den pH innerhalb von einer Stunde mit 10%iger Natriumcarbonatlösung auf pH 6,0, lässt die Temperatur auf Raumtemperatur ansteigen und rührt weitere 2 Stunden bei pH 6 nach. Die entstandene dunkelblaue Lösung wird umkehrosmotisiert und gefriergetrocknet. Man erhält einen Farbstoff, der in Form der freien Säure der Verbindung der Formel

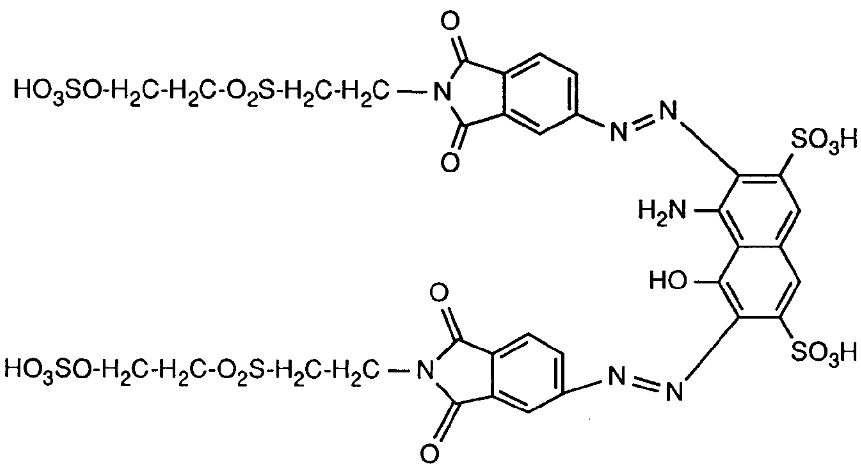

entspricht und Baumwolle und Wolle in marineblauen Tönen mit guten Allgemeinechtheiten färbt.

Beispiel 16:

Behandelt man die gemäss Beispiel 15 erhaltene Farbstofflösung in einem alkalischen Medium wie in Beispiel 14 beschrieben, so erhält man nach der Umkehrosmose und dem Gefriertrocknen einen Farbstoff, welcher in der Form der freien Säure dem Isomerengemisch im Verhältnis 1:1:1:1 der Formel

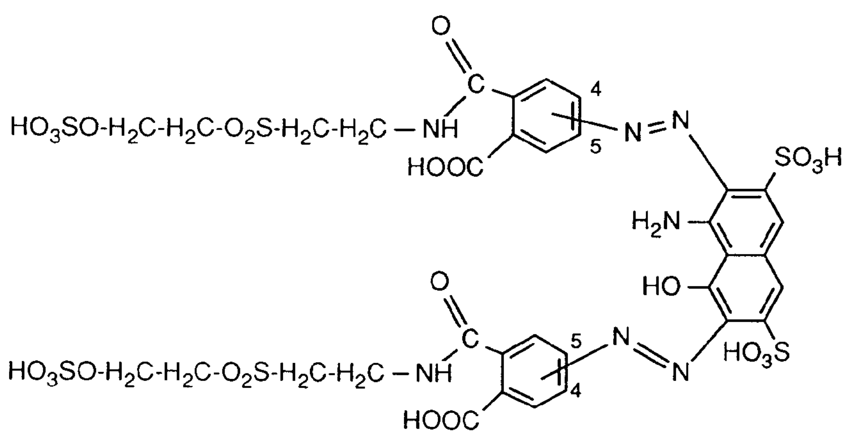

entspricht und Baumwolle und Wolle in marineblauen Tönen mit guten Allgemeinechtheiten färbt.

Beispiel 17:

Man lässt eine mit 10%iger Natriumcarbonatlösung auf pH 6 gestellte Lösung von 31,9 Teilen 1-Amino-8-hydroxynaphthalin-3,6-disulfosäure in 100 Teilen Wasser unter intensivem Rühren innerhalb von einer Stunde zu einer eiskalten Dispersion aus 9,3 Teilen Cyanurchlorid, 0,05 Teilen eines Netzmittels, 0,1 Teile Dinatriumhydrogenphosphat und 100 Teilen Eiswasser zulaufen und rührt eine Stunde bei 0 - 5°C nach. Danach lässt man die Temperatur auf Raumtemperatur ansteigen, erhöht gleichzeitig den pH mit 10%iger Natriumcarbonatlösung langsam auf pH 6 und rührt eine Stunde nach. Man erhält eine Kupplungskomponente, die in der Form der freien Säure der Formel

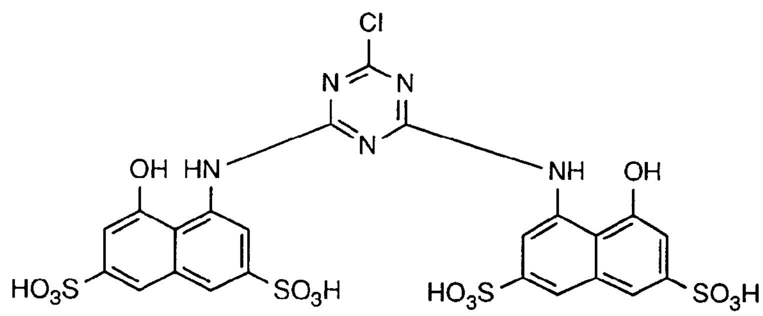

entspricht. Zu dieser Suspension lässt man bei 0 - 5°C die gemäss Beispiel 13, 1. Absatz hergestellte Diazosuspension zulaufen und hält dabei den pH mit 10%iger Natriumcarbonatlösung bei pH 6,0 - 6,5. Man rührt eine Stunde bei 0 - 5°C und weitere 2 Stunden bei Raumtemperatur und einem pH von 6,5 nach. Nach vollständiger Kupplung wird die rote Lösung umkehrosmotisiert und gefriergetrocknet. Man erhält einen Farbstoff, der in der Form der freien Säure der Formel

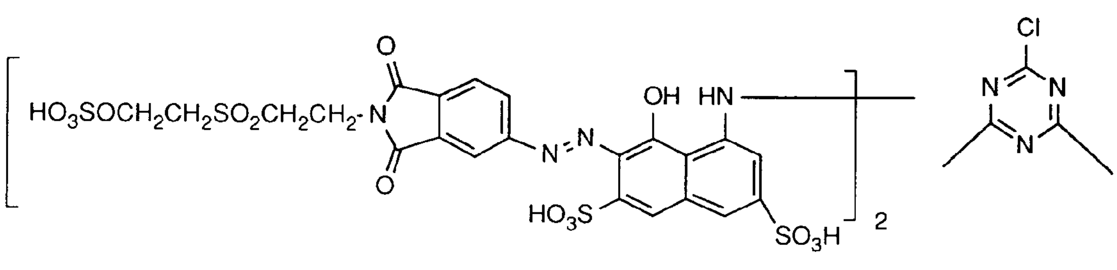

entspricht und Baumwolle und Wolle in brillanten roten Tönen mit guten Allgemeinechtheiten färbt.

Beispiel 18:

Behandelt man die gemäss Beispiel 17 erhaltene Farbstofflösung in einem alkalischen Medium wie in Beispiel 14 beschrieben, so erhält man nach der Umkehrosmose und dem Gefriertrocknen einen Farbstoff, welcher in der Form der freien Säure dem Isomerengemisch im Verhältnis 1:2:1 der Formel

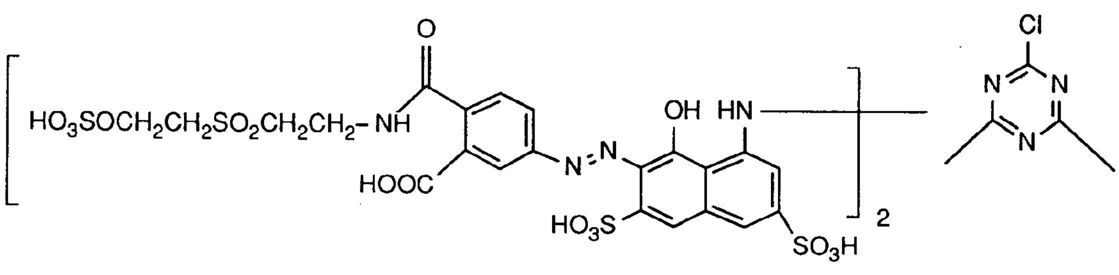

entspricht. Er besitzt ähnliche Eigenschaften wie der im Beispiel 17 beschriebene Farbstoff.

Beispiele 19-35 :

Setzt man die Diazokomponente der Formel

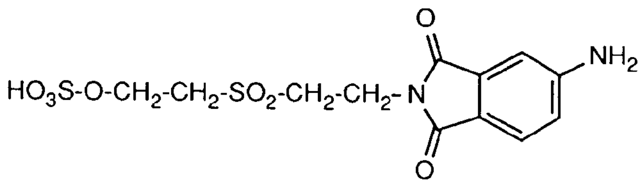

analog wie in den Beispielen 13, 15 oder 17 beschrieben mit den in der folgenden Tabelle angegebenen Kupplungskomponenten um, erhält man analoge Farbstoffe; hierbei markieren die an den Kupplungskomponenten angebrachten Pfeile die Kupplungsposition bzw. Kupplungspositionen:

Tabelle:

19

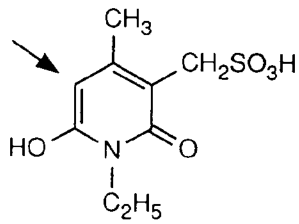

20

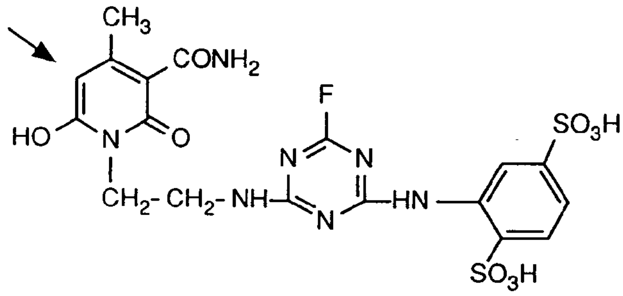

21

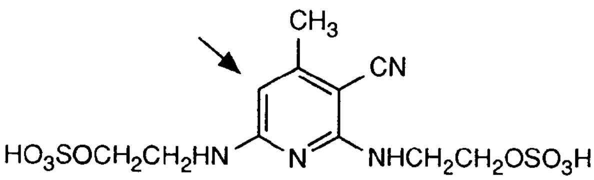

22

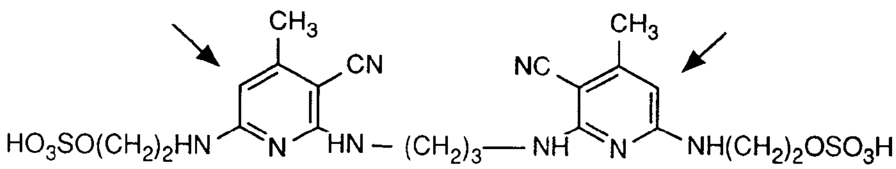

23

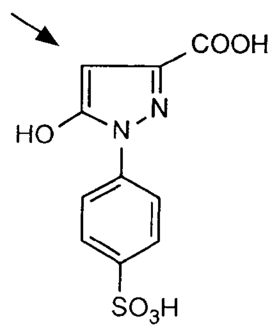

24

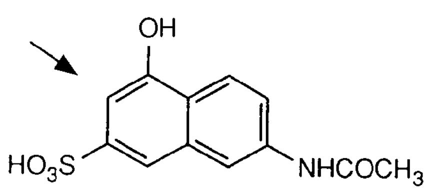

25

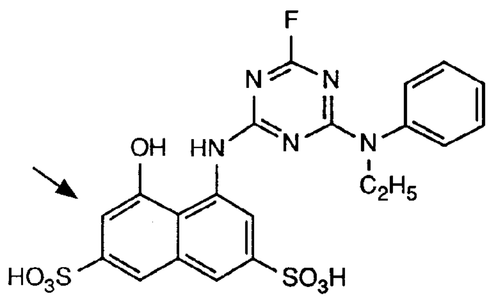

26

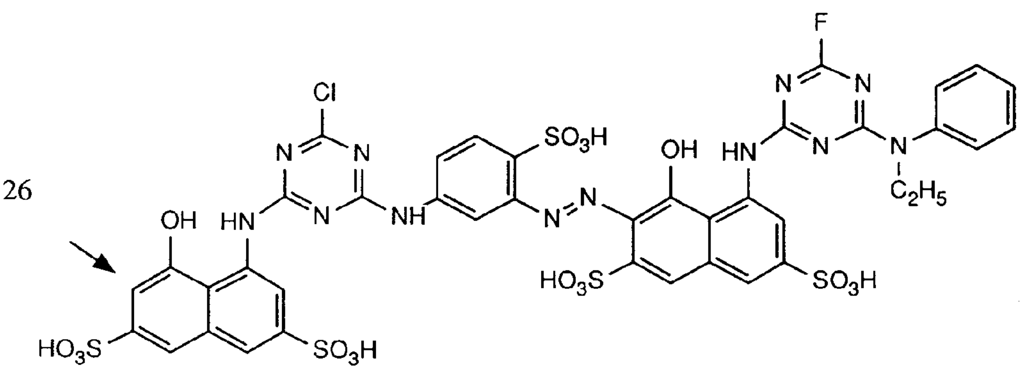

27

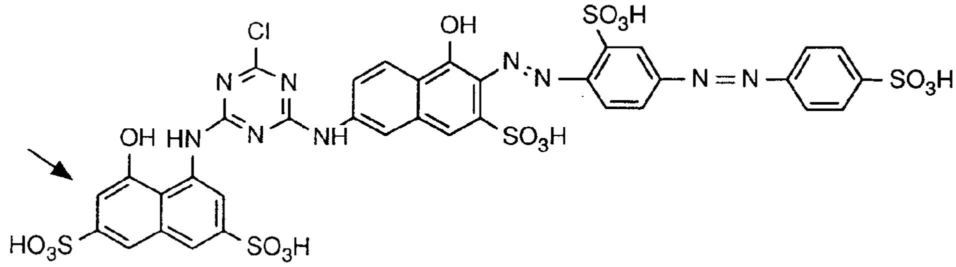

28

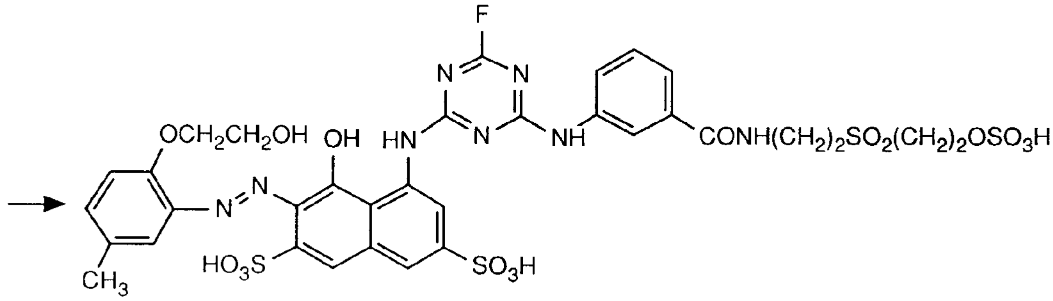

29

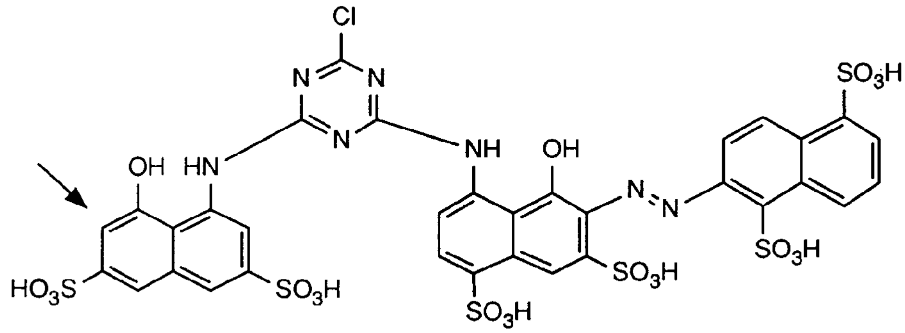

30

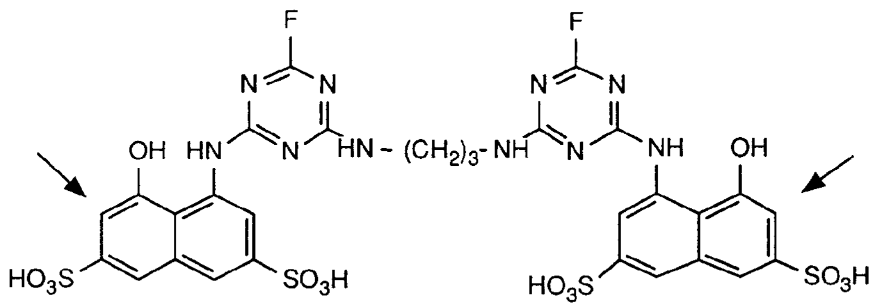

31

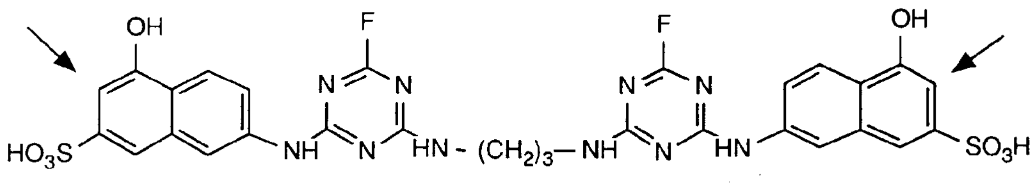

32

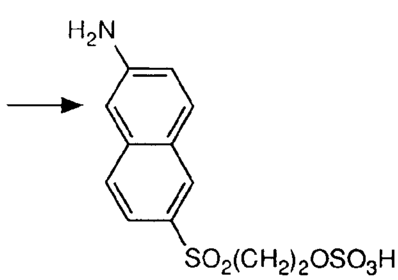

33

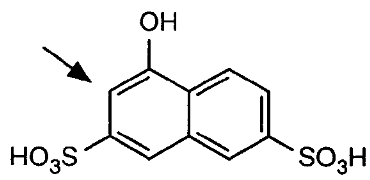

34

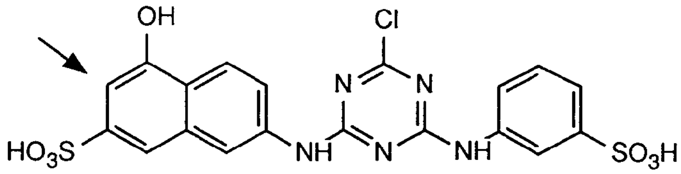

35

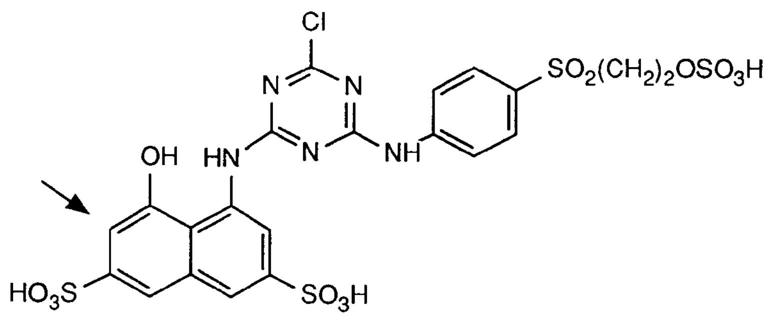

Beispiele 36-40:

Verfährt man wie in den Beispielen 13 bis 35 beschrieben, und verwendet anstelle der Diazokomponente gemäss Beispiel 3 die äquivalente Menge der folgenden Diazokomponenten, werden analoge Farbstoffe mit ähnlich guten Allgemeinechtheiten erhalten:

36

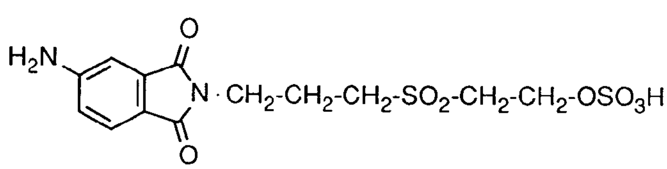

37

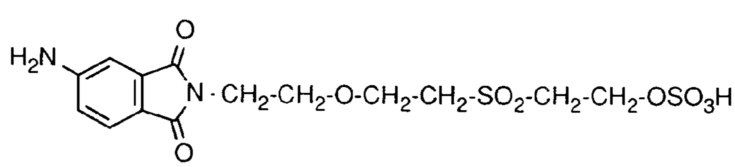

38 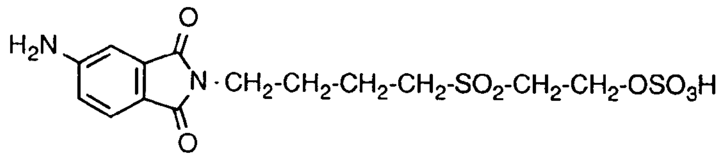

39 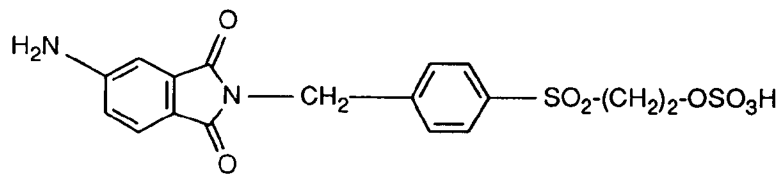

40 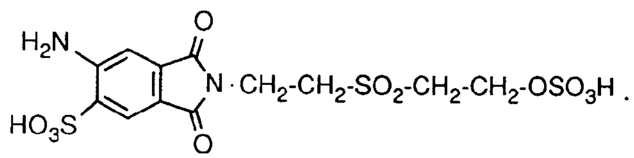

Färbevorschrift I

2 Teile des gemäss Beispiel 13 erhaltenen Farbstoffes werden in 400 Teilen Wasser gelöst; dazu gibt man 1500 Teile einer Lösung, die pro Liter 53 g Natriumchlorid enthält. In dieses Färbebad geht man bei 40°C mit 100 Teilen Baumwollgewebe ein. Nach 45 Minuten werden 100 Teile einer Lösung, die pro Liter 16 g Natriumhydroxyd und 20 g kalziniertes Soda enthält, zugegeben. Die Temperatur des Färbebades wird weitere 45 Minuten bei 40°C gehalten. Danach wird die gefärbte Ware gespült, während einer Viertelstunde mit einem nichtionogenen Waschmittel kochend geseift, nochmals gespült und getrocknet.

Färbevorschrift II

2 Teile des gemäss Beispiel 13 erhaltenen Reaktivfarbstoffes werden in 400 Teilen Wasser gelöst; dazu gibt man 1500 Teile einer Lösung die pro Liter 53 g Natriumchlorid enthält. In dieses Färbebad geht man bei 35°C mit 100 Teilen eines Baumwollgewebes ein. Nach 20 Minuten werden 100 Teile einer Lösung, die pro Liter 16 g Natriumhydroxyd und 20 g kalziniertes Soda enthält, zugegeben. Die Temperatur des Färbebades wird weitere 15 Minuten bei 35°C gehalten. Danach wird die Temperatur innerhalb von 20 Minuten auf 60°C erhöht. Die Temperatur wird weitere 35 Minuten bei 60°C gehalten. Danach wird gespült, während einer Viertelstunde mit einem nichtionogenen Waschmittel kochend geseift, nochmals gespült und getrocknet.

Färbevorschrift III

8 Teile des gemäss Beispiel 13 erhaltenen Reaktivfarbstoffes werden in 400 Teilen Wasser gelöst; dazu gibt man 1400 Teile einer Lösung, die pro Liter 100 g Natriumsulfat enthält. In dieses Färbebad geht man bei 25°C mit 100 Teilen eines Baumwollgewebes ein. Nach 10 Minuten werden 200 Teile einer Lösung, die pro Liter 150 g Trinatriumphosphat enthält, zugegeben. Danach wird die Temperatur des Färbebades innerhalb von 10 Minuten auf 60°C erhöht. Die Temperatur wird weitere 90 Minuten auf 60°C gehalten. Danach wird gespült, während einer Viertelstunde mit einem nichtionogenen Waschmittel kochend geseift, nochmals gespült und getrocknet.

Färbevorschrift IV

4 Teile des gemäss Beispiel 13 erhaltenen Reaktivfarbstoffes werden in 50 Teilen Wasser gelöst. Dazu gibt man 50 Teile einer Lösung, die pro Liter 5 g Natriumhydroxyd und 20 g kalziniertes Soda enthält. Mit der erhaltenen Lösung wird ein Baumwollgewebe foulardiert, so dass es um 70 % seines Gewichts zunimmt, und

dann auf eine Kaule aufgewickelt. Das Baumwollgewebe wird so während 3 Stunden bei Raumtemperatur gelagert. Danach wird die gefärbte Ware gespült, während einer Viertelstunde mit einem nichtionogenen Waschmittel kochend geseift, nochmals gespült und getrocknet.

Färbevorschrift V

6 Teile des gemäss Beispiel 13 erhaltenen Reaktivfarbstoffes werden in 50 Teilen Wasser gelöst. Dazu gibt man 50 Teile einer Lösung, die pro Liter 16 g Natriumhydroxyd und 0,04 Liter Wasserglas (38°bé) enthält. Mit der erhaltenen Lösung wird ein Baumwollgewebe foulardiert, so dass es um 70 % seines Gewichts zunimmt, und dann auf eine Kaule aufgewickelt. Das Baumwollgewebe wird so während 10 Stunden bei Raumtemperatur gelagert. Danach wird die gefärbte Ware gespült, während einer Viertelstunde mit einem nichtionogenen Waschmittel kochend geseift, nochmals gespült und getrocknet.

Färbevorschrift VI

2 Teile des gemäss Beispiel 13 erhaltenen Reaktivfarbstoffes werden unter Zusatz von 0,5 Teilen m-nitrobenzolsulfonsaurem Natrium in 100 Teilen Wasser gelöst. Mit der erhaltenen Lösung wird ein Baumwollgewebe imprägniert, so dass es um 75 % seines Gewichts zunimmt, und dann getrocknet. Dann imprägniert man das Gewebe mit einer 20°C warmen Lösung, die pro Liter 4 g Natriumhydroxyd und 300 g Natriumchlorid enthält, quetscht auf 75 % Gewichtszunahme ab, dämpft die Färbung während 30 Sekunden bei 100 bis 102°C, spült, seift während einer Viertelstunde in einer 0,3%igen kochenden Lösung eines nichtionogenen Waschmittels, spült und trocknet.

Färbevorschrift VII

0,1 Teile Farbstoff gemäss Beispiel 13 werden in 200 Teilen entmineralisiertem Wasser gelöst und 0,5 Teile Glaubersalz, 0,1 Teile eines Egalisiermittels sowie 0,5 Teile Natriumacetat zugegeben. Dann wird mit 80%iger Essigsäure auf pH 5,5 eingestellt. Das Färbebad wird 10 Minuten auf 50°C erwärmt und dann 10 Teile eines Wollgewebes zugegeben. Man erwärmt innerhalb von ca. 50 Minuten auf 100°C und färbt 60 Minuten bei dieser Temperatur. Danach lässt man auf 90°C abkühlen und entnimmt das Färbegut. Das Wollgewebe wird mit warmem und kaltem Wasser gespült, anschliessend geschleudert und getrocknet. Es wird eine brillante rote Färbung erhalten, die sehr gute Licht- und Nassechtheiten aufweist.

Druckvorschrift I

3 Teile des gemäss Beispiel 13 erhaltenen Reaktivfarbstoffes werden unter schnellem Rühren in 100 Teile einer Stammverdickung, enthaltend 50 Teile 5%ige Natriumalginatverdickung, 27,8 Teile Wasser, 20 Teile Harnstoff, 1 Teil m-nitrobenzolsulfonsaures Natrium sowie 1,2 Teile Natriumhydrogencarbonat, eingestreut. Mit der so erhaltenen Druckpaste bedruckt man ein Baumwollgewebe, trocknet und dämpft den erhaltenen bedruckten Stoff 2 Minuten bei 102°C in gesättigtem Dampf. Das bedruckte Gewebe wird dann gespült, gegebenenfalls kochend geseift und nochmals gespült, und anschliessend getrocknet.

Druckvorschrift II

5 Teile des gemäss Beispiel 13 erhaltenen Reaktivfarbstoffes werden unter schnellem Rühren in 100 Teile einer Stammverdickung, enthaltend 50 Teile 5%ige Natriumalginatverdickung, 36,5 Teile Wasser, 10 Teile Harnstoff, 1 Teil m-nitrobenzolsulfonsaures Natrium sowie 2,5 Teile Natriumhydrogencarbonat, eingestreut. Mit der so erhaltenen Druckpaste, deren Stabilität den technischen Anforderungen entspricht, bedruckt man ein Baumwollgewebe, trocknet und dämpft den erhaltenen bedruckten Stoff 8 Minuten bei 102°C in gesättigtem Dampf. Das bedruckte Gewebe wird dann gespült, gegebenenfalls kochend geseift und nochmals gespült, und anschliessend getrocknet.

**Patentansprüche**

1. Verbindungen der Formel

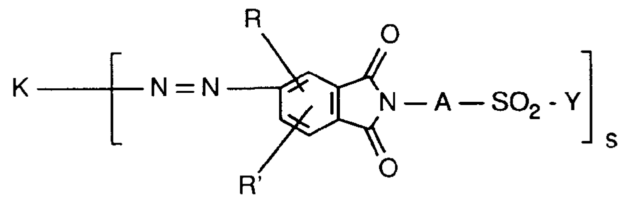

(1),

worin
K der Rest einer Kupplungskomponente ist,
s für die Zahl 1 oder 2 steht,
R und R' unabhängig voneinander je Wasserstoff, Sulfo, Hydroxy, $C_1$-$C_4$-Alkoxy, Halogen oder Cyano bedeuten,
A unsubstituiertes oder durch Halogen, Hydroxy, Sulfato, Carboxy, Cyano, $C_2$-$C_4$-Alkanoyloxy, $C_1$-$C_4$-Alkoxycarbonyl oder Carbamoyl substituiertes oder durch eine Gruppe -O- oder -NR"- unterbrochenes $C_1$-$C_6$-Alkylen oder unsubstituiertes oder im Phenylenteil durch $C_1$-$C_4$-Alkyl, $C_1$-$C_4$-Alkoxy, Halogen oder Sulfo substituiertes $C_1$-$C_6$-Alkylenphenylen ist,
R" für Wasserstoff oder $C_1$-$C_4$-Alkyl steht,
Y Vinyl oder einen Rest $-CH_2-CH_2-U$ bedeutet, und
U eine alkalisch abspaltbare Abgangsgruppe ist.

**2.** Verbindungen gemäss Anspruch 1, dadurch gekennzeichnet, dass R Wasserstoff, Sulfo, Chlor, Brom oder Cyano und R' Wasserstoff sind.

**3.** Verbindungen gemäss Anspruch 1 oder 2, dadurch gekennzeichnet, dass A einen unsubstituierten $C_1$-$C_6$-Alkylenrest oder einen Rest der Formel

$$-(CH_2)_p-O-(CH_2)_q-$$

oder

$$-(CH_2)_p-NH-(CH_2)_q-,$$

worin p und q unabhängig voneinander je eine ganze Zahl von 1 bis 6 darstellen, bedeutet.

**4.** Verbindungen gemäss einem der Ansprüche 1 bis 3, dadurch gekennzeichnet, dass A für $C_2$-$C_4$-Alkylen oder für einen Rest der Formel $-(CH_2)_{2-4}-O-(CH_2)_{2-4}-$ oder $-(CH_2)_{2-4}-NH-(CH_2)_{2-4}-$ steht.

**5.** Verbindungen gemäss einem der Ansprüche 1 bis 4, dadurch gekennzeichnet, dass A für 1,2-Ethylen, 1,3-Propylen, 1,4-Butylen oder einen Rest der Formel

$$-(CH_2)_{2-3}-O-(CH_2)_{2-3}-$$

oder

$$-(CH_2)_{2-3}-NH-(CH_2)_{2-3}-$$

steht.

**6.** Verbindungen gemäss einem der Ansprüche 1 bis 5, dadurch gekennzeichnet, dass Y Vinyl, β-Brom- oder β-Chlorethyl, β-Acetoxyethyl, β-Benzoyloxyethyl, β-Phosphatoethyl, β-Sulfatoethyl oder β-Thiosulfatoethyl und bevorzugt Vinyl oder β-Sulfatoethyl bedeutet.

**7.** Verbindungen der Formel (1) gemäss Anspruch 1, dadurch gekennzeichnet, dass R Wasserstoff oder Sulfo, R' Wasserstoff, A $C_2$-$C_4$-Alkylen oder einen Rest der Formel $-(CH_2)_{2-4}-O-(cH_2)_{2-4}-$ oder $-(CH_2)_{2-4}-NH-(CH_2)_{2-4}-$, und Y Vinyl oder β-Sulfatoethyl bedeuten.

**8.** Verbindungen gemäss einem der Ansprüche 1 bis 7, dadurch gekennzeichnet, dass K den Rest einer Kupplungskomponente der Benzol-, Naphthalin-, Naphthylazobenzol-, Phenylazonaphthalin-, 4-Alkyl-6-hydroxypyridon-(2)-, 2,5-Diamino-4-alkylpyridin-, 1-Arylpyrazolon-(5)- oder 1-Aryl-5-aminopyrazol-Reihe ist, welche farbstoffübliche Substituenten und gegebenenfalls eine oder mehrere faserreaktive Gruppen enthält.

**9.** Verbindungen gemäss einem der Ansprüche 1 bis 8, dadurch gekennzeichnet, dass K einen oder mehrere gleiche oder verschiedene Substituenten aus der Gruppe $C_1$-$C_{12}$-Alkyl, $C_1$-$C_8$-Alkoxy, im Alkylteil durch Hydroxy, $C_1$-$C_4$-Alkoxy oder Sulfato substituiertes $C_1$-$C_4$-Alkoxy, $C_2$-$C_8$-Acylamino, Amino, gegebenen-

falls im Alkylteil durch Hydroxy, Sulfo, Sulfato oder $C_1$-$C_4$-Alkoxy substituiertes N-Mono- oder N,N-Di-$C_1$-$C_4$-Alkylamino, gegebenenfalls im Phenylteil durch Methyl, Methoxy, Halogen oder Sulfo substituiertes Phenylamino, gegebenenfalls im Alkylteil durch Hydroxy, Sulfo oder Sulfato oder im Phenylteil gegebenenfalls durch Methyl, Methoxy, Halogen oder Sulfo substituiertes N-$C_1$-$C_4$-Alkyl-N-phenylamino, gegebenenfalls durch Sulfo substituiertes Naphthylamino, $C_2$-$C_8$-Alkanoyl, Benzoyl, $C_1$-$C_4$-Alkoxycarbonyl, $C_1$-$C_4$-Alkylsulfonyl, Phenyl- oder Naphthylsulfonyl, Trifluormethyl, Nitro, Cyano, Hydroxyl, Halogen, Carbamoyl, N-$C_1$-$C_4$-Alkylcarbamoyl, Sulfamoyl, N-$C_1$-$C_4$-Alkyl-sulfamoyl, N-(β-Hydroxyethyl)-sulfamoyl, N,N-Di-(β-hydroxyethyl)-sulfamoyl, N-Phenylsulfamoyl, Ureido, Carboxy, Sulfomethyl, Sulfo oder Sulfato und faserreaktiver Rest der Formel

$$-SO_2-Y \quad (2a),$$

$$-CONR_1-(CH_2)_n-SO_2-Y \quad (2b)$$

oder

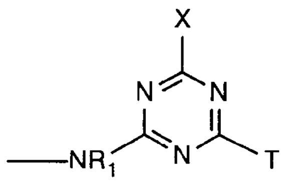

(2c) trägt,

worin X Halogen, 3-Carboxypyndin-1-yl oder 3-Carbamoylpyridin-1-yl bedeutet, T unabhängig die Bedeutung von X hat oder für Hydroxy, $C_1$-$C_4$-Alkoxy, Phenoxy, $C_1$-$C_4$-Alkylthio, Morpholino, gegebenenfalls durch nicht-reaktive Reste substituiertes Amino oder für einen Reaktivrest der Formel

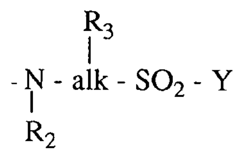

(3a),

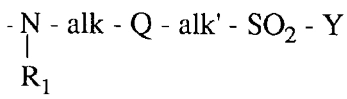

(3b),

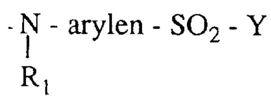

(3c),

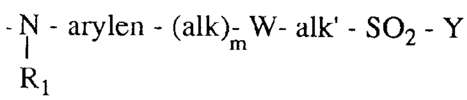

(3d),

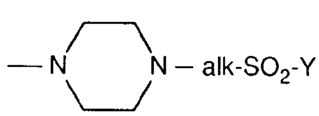

(3e)

oder

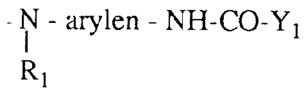 (3f)

steht,
$R_1$ Wasserstoff oder $C_1$-$C_4$-Alkyl ist,
$R_2$ Wasserstoff, gegebenenfalls durch Hydroxy, Sulfo, Sulfato, Carboxy oder Cyano substituiertes $C_1$-$C_4$-Alkyl oder einen Rest

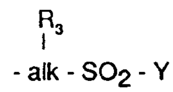

bedeutet,
$R_3$ Wasserstoff, Hydroxy, Sulfo, Sulfato, Carboxy, Cyano, Halogen, $C_1$-$C_4$-Alkoxycarbonyl, $C_1$-$C_4$-Alkanoyloxy, Carbamoyl oder die Gruppe -$SO_2$-Y ist, alk und alk' unabhängig voneinander $C_1$-$C_6$-Alkylen sind, arylen einen unsubstituierten oder durch Sulfo, Carboxy, $C_1$-$C_4$-Alkyl, $C_1$-$C_4$-Alkoxy oder Halogen substituierten Phenylen- oder Naphthylenrest bedeutet,
$Y_1$ für eine Gruppe -$CHX_2$-$CH_2X_2$ oder -$CX_2$=$CH_2$ steht und $X_2$ Chlor oder Brom bedeutet,
Q ein Rest -O- oder -NR", worin R" die im Anspruch 1 angegebene Bedeutung hat- ist,
W für eine Gruppe -$SO_2$-$NR_2$-, -$CONR_2$- oder -$NR_2CO$- steht,
m die Zahl 0 oder 1 ist,
n für eine ganze Zahl von 1 bis 6 steht und Y die im Anspruch 1 genannte Bedeutung hat.

**10.** Verbindungen gemäss einem der Ansprüche 1 bis 9, dadurch gekennzeichnet, dass s die Zahl 1 bedeutet und K für einen Rest der Formel

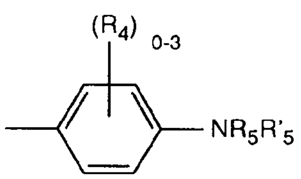 (4a)

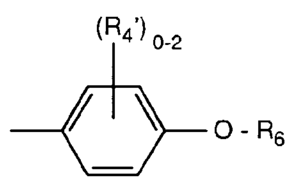 (4b)

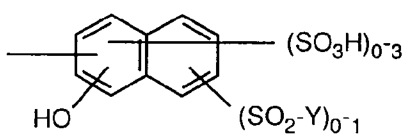 (4c)

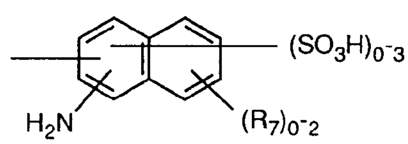 (4d)

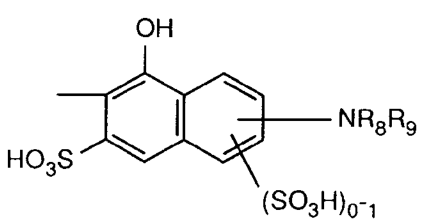 (4e)

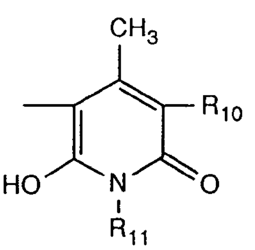 (4f)

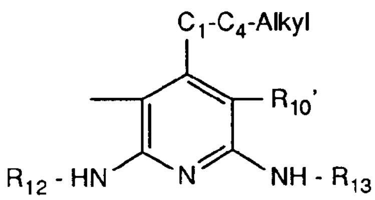 (4g) 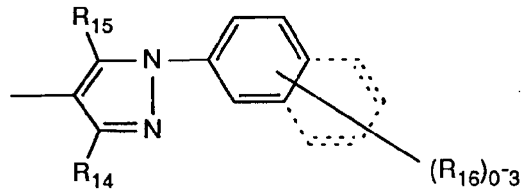 (4h)

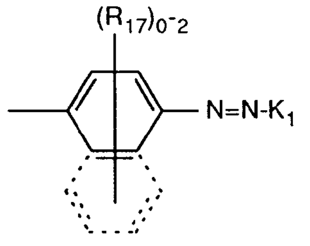 (4i) 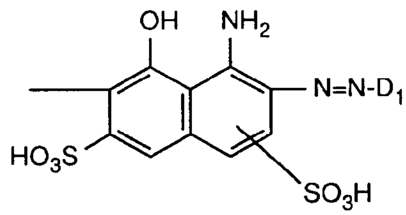 (4j)

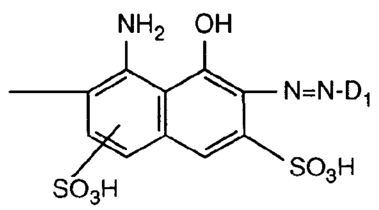 (4k), oder

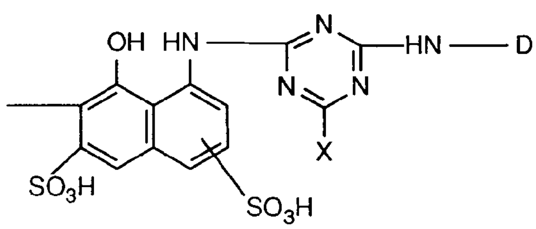 (4l) steht

worin

$(R_4)_{0-3}$ für 0 bis 3 gleiche oder verschiedene Reste $R_4$ z.B. aus der Gruppe $C_1$-$C_4$-Alkyl, $C_1$-$C_4$-Alkoxy, im Alkylteil durch Hydroxy, $C_1$-$C_4$-Alkoxy oder Sulfato substituiertes $C_1$-$C_4$-Alkoxy, Halogen, Carboxy, Hydroxy, Amino, N-Mono- oder N,N-Di-$C_1$-$C_2$-Alkylamino, $C_2$-$C_4$-Alkanoylamino, Ureido, Sulfo, $C_1$-$C_4$-Alkylsulfonyloxy, $C_1$-$C_4$-Alkylsulfonylamino oder einen Reaktivrest der im Anspruch 9 angegebenen Formel (2c) steht;

$(R_4')_{0-2}$ für 0 bis 2 gleiche oder verschiedene Reste $R_4'$ aus der Gruppe $C_1$-$C_4$-Alkyl, $C_1$-$C_4$-Alkoxy, Phenoxy, Halogen, Sulfo und Hydroxy steht;

$R_5$ Wasserstoff oder $C_1$-$C_4$-Alkyl ist;

$R_5'$ unabhängig die Bedeutung von $R_5$ hat oder für $C_2$-$C_4$-Alkanoyl, $C_1$-$C_4$-Hydroxyalkyl, $C_1$-$C_4$-Sulfatoalkyl, $C_1$-$C_4$-Alkoxyalkyl oder gegebenenfalls durch Methyl, Methoxy, Chlor oder Sulfo substituiertes Phenyl steht;

$R_6$ Wasserstoff, $C_1$-$C_4$-Alkyl oder Phenyl bedeutet;

$(R_7)_{0-2}$ für 0 bis 2 gleiche oder verschiedene Substituenten aus der Gruppe Hydroxy und faserreaktiver Rest der im Anspruch 9 angegebenen Formel (2a) steht;

$R_8$ Wasserstoff oder gegebenenfalls durch Hydroxy, $C_1$-$C_4$-Alkoxy, Sulfato, Sulfo, Halogen oder Cyano substituiertes $C_1$-$C_4$-Alkyl ist;

$R_9$ unabhängig die Bedeutung von $R_8$ hat oder $C_2$-$C_4$-Alkanoyl, Benzoyl oder einen faserreaktiven Rest der im Anspruch 9 angegebenen Formel (2c) bedeutet;

$R_{10}$ und $R_{10}'$ unabhängig voneinander je Carbamoyl, Sulfomethyl oder Cyano sind;

$R_{11}$ für Wasserstoff oder $C_1$-$C_4$-Alkyl, welches gegebenenfalls durch einen Rest der Formel (2c) substi-

tuiert ist, steht;

$R_{12}$ und $R_{13}$ unabhängig voneinander je für Wasserstoff oder gegebenenfalls durch Hydroxy, $C_1$-$C_4$-Alkoxy, Phenoxy, Sulfato, Sulfo, Carboxy, Amino oder N-Mono- oder N,N-Di-$C_1$-$C_4$-Alkylamino, welches seinerseits im Alkylteil durch Hydroxy, $C_1$-$C_4$-Alkoxy, Phenoxy, Sulfato, Sulfo, Carboxy oder einen faserreaktiven Rest der zuvor angegebenen Formel (2c) substituiert sein kann, substituiertes und/oder mit Ausnahme von Methyl gegebenenfalls durch -O- unterbrochenes $C_1$-$C_{12}$-Alkyl stehen;

$R_{14}$ Methyl oder Carboxy bedeutet;

$R_{15}$ Hydroxy oder Amino ist;

$(R_{16})_{0-3}$ für 0 bis 3 gleiche oder verschiedene Reste $R_{16}$ aus der Gruppe Sulfo, Halogen, Hydroxy, $C_1$-$C_4$-Alkoxy und $C_1$-$C_4$-Alkyl steht;

$(R_{17})_{0-2}$ für 0 bis 2 gleiche oder verschiedene Reste $R_{17}$ aus der Gruppe $C_1$-$C_4$-Alkyl, $C_1$-$C_4$-Alkoxy, im Alkylteil durch Hydroxy, $C_1$-$C_4$-Alkoxy oder Sulfato substituiertes $C_1$-$C_4$-Alkoxy, $C_2$-$C_4$-Alkanoylamino, Ureido, Halogen und Sulfo steht;

$K_1$ unabhängig einen Rest der oben angegebenen Formel (4a) - (4h) oder (4j) - (41) bedeutet;

$D_1$ ein Phenyl- oder 1- oder 2-Naphthylrest ist, der 1 bis 3 gleiche oder verschiedene Reste aus der Gruppe Sulfo, $C_1$-$C_4$-Alkyl, $C_1$-$C_4$-Alkoxy, Halogen oder faserreaktiver Rest der im Anspruch 9 angegebenen Formel (2a) oder (2b) trägt;

D der Rest eines Monoazo- oder Disazochromophors ist;

und X die im Anspruch 9 und Y die im Anspruch 1 angegebene Bedeutung haben.

**11.** Verbindungen gemäss einem der Ansprüche 1 bis 10, dadurch gekennzeichnet, dass s die Zahl 1 bedeutet und K für einen Rest der Formel

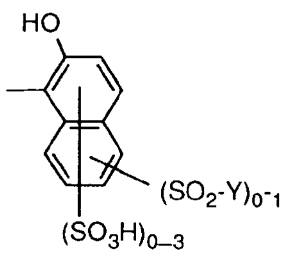 (4c'), 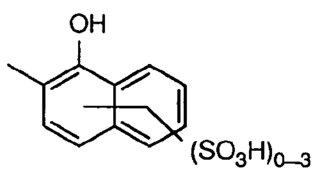 (4c''),

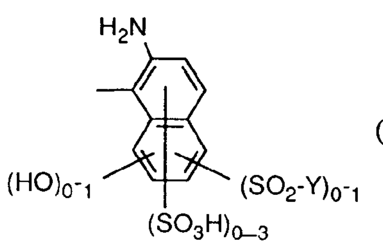 (4d'), 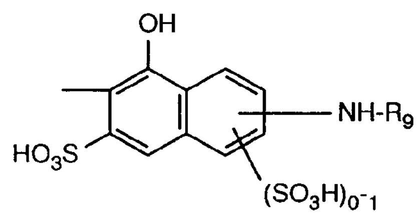 , (4e')

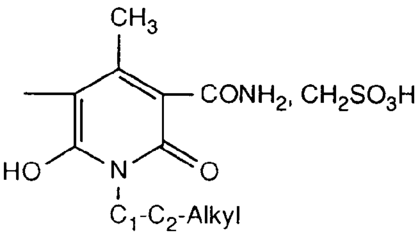 (4f'), 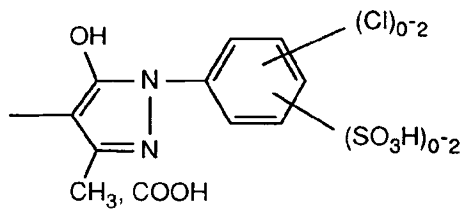 (4h'),

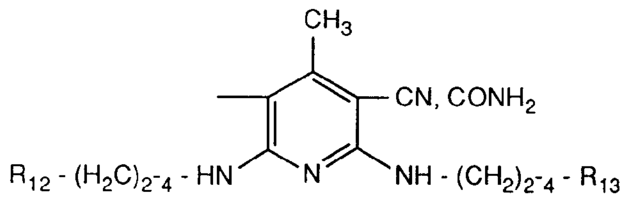 (4g'),

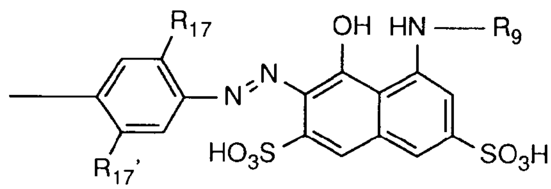 (4i'),

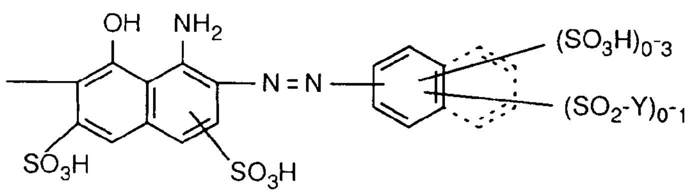 (4j')

oder

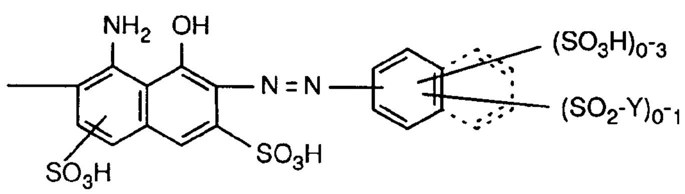 (4k') steht,

worin $R_9$ Acetyl, Benzoyl oder einen Rest der im Anspruch 9 angegebenen Formel (2c) bedeutet, $R_{12}$ Wasserstoff, Hydroxy oder Sulfato ist, $R_{13}$ unabhängig die Bedeutung von $R_{12}$ hat oder für einen Rest der im Anspruch 9 angegebenen Formel (2c) steht, $R_{17}$ und $R_{17}'$ unabhängig voneinander je für Wasserstoff, Methyl, Methoxy, 2-Hydroxyethoxy, 2-Sulfatoethoxy, Acetylamino, Propionylamino, Ureido oder Sulfo stehen, und Y die im Anspruch 1 angegebene Bedeutung hat.

12. Verbindungen gemäss einem der Ansprüche 1 bis 9, dadurch gekennzeichnet, dass s die Zahl 2 bedeutet und K der Rest einer doppelt ankuppelbaren Kupplungskomponente der Aminonaphtholsulfonsäure-Reihe oder ein Rest der Formel

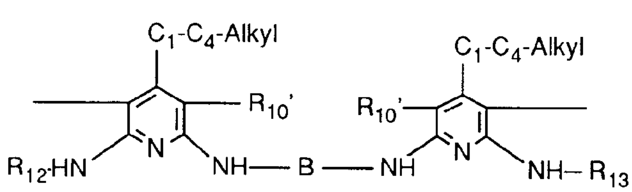 (6)

oder

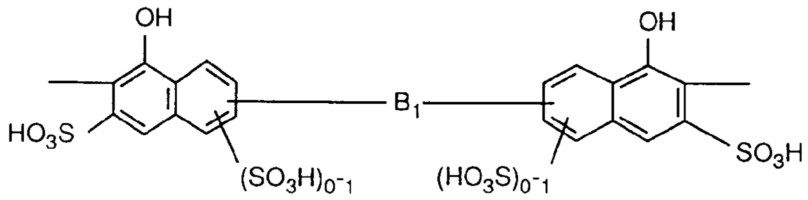

(7) ist,

worin $B_1$ ein Brückenglied der Formel

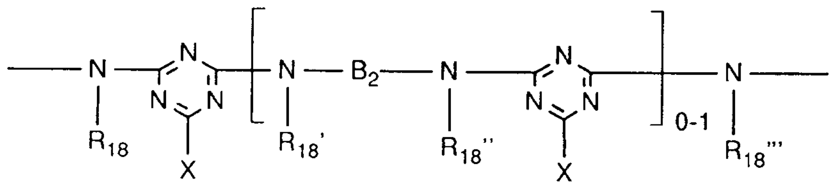

(8),

bedeutet, $R_{18}$, $R_{18}'$, $R_{18}''$ und $R_{18}'''$ unabhängig voneinander je Wasserstoff oder unsubstituiertes oder z.B. durch Halogen, Hydroxy, Cyano, $C_1$-$C_4$-Alkoxy, Carboxyl, Sulfo oder Sulfato substituiertes $C_1$-$C_4$-Alkyl sind, $B_2$ ein aliphatisches, cycloaliphatisches oder aromatisches Brückenglied bedeutet und $R_{10}'$, $R_{12}'$ $R_{13}$ jeweils die im Anspruch 10 und X die im Anspruch 9 angegebene Bedeutung hat.

13. Verbindungen gemäss einem der Ansprüche 1 bis 9 oder 12, dadurch gekennzeichnet, dass s die Zahl 2 bedeutet und K ein Rest der Formel

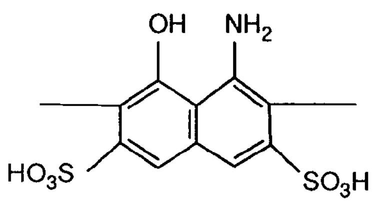

(11a) (Rest von H-Säure),

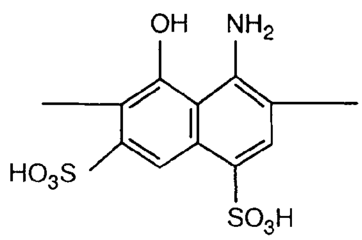

(11b) (Rest von K-Säure),

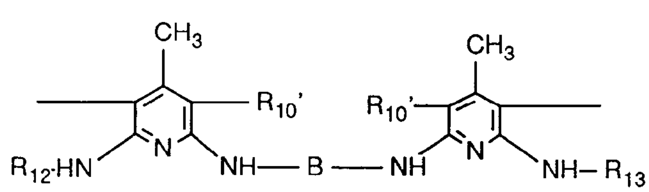

(6a),

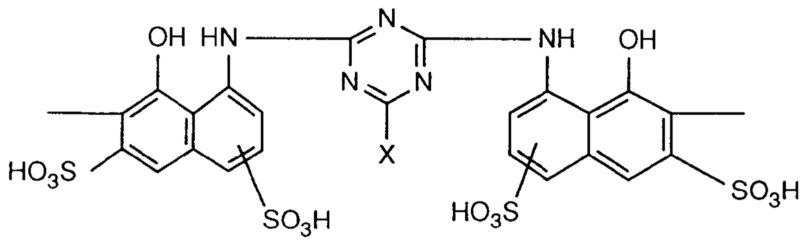

(7a),

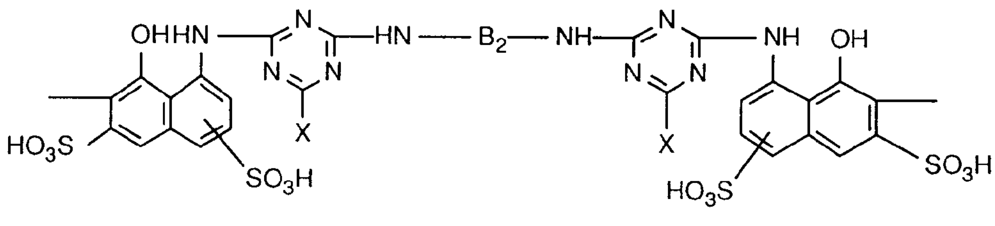

(7b),

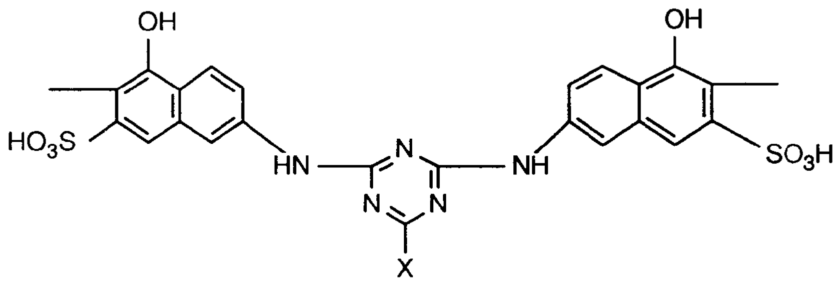

(7c)

oder

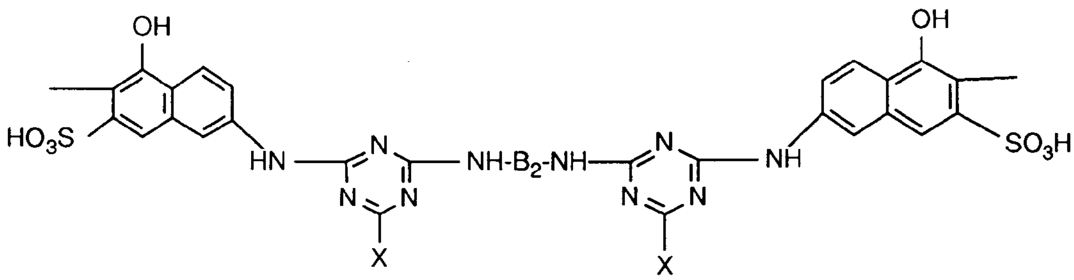

(7d) ist,

worin $R_{10}$' Cyano oder Carbamoyl ist, $R_{12}$ und $R_{13}$ unabhängig voneinander jeweils für Wasserstoff oder gegebenenfalls durch Hydroxy, Methoxy, Ethoxy, Sulfato oder Sulfo, substituiertes $C_1$-$C_6$-Alkyl stehen, B geradkettiges oder verzweigtes $C_2$-$C_6$-Alkylen, welches gegebenenfalls durch Hydroxy, Sulfo oder Sulfato substituiert sein kann, bedeutet, $B_2$ unabhängig die Bedeutung von B hat oder für unsubstituiertes oder durch Sulfo, Methyl oder Methoxy substituiertes 1,3- oder 1,4-Phenylen, durch 1 oder 2 Sulfogruppen substituiertes Naphthylen oder für einen Rest der Formel

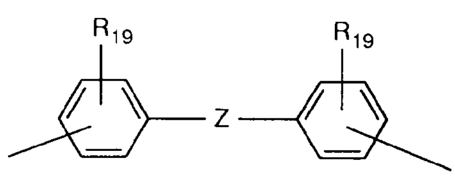

(10a) oder

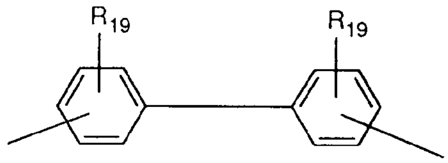

(10b),

worin Z -NHCONH-, -O-, -NH-, -CH=CH- oder $-CH_2-$ und $R_{19}$ Wasserstoff oder Sulfo bedeuten, steht, und X die im Anspruch 9 genannte Bedeutung hat.

**14.** Verbindungen gemäss Anspruch 13, dadurch gekennzeichnet, dass s die Zahl 2 bedeutet und K ein Rest der Formel (11a), (7a), (7b), (7c) oder (7d), worin $B_2$ geradkettiges oder verzweigtes $C_2$-$C_6$-Alkylen, welches unsubstituiert oder durch Hydroxy, Sulfo oder Sulfato substituiert ist, bedeutet und X für Chlor oder Fluor steht.

**15.** Verfahren zur Herstellung von Verbindungen der Formel (1) gemäss Anspruch 1, dadurch gekennzeichnet, dass man in etwa s Moläquivalente der Verbindung der Formel

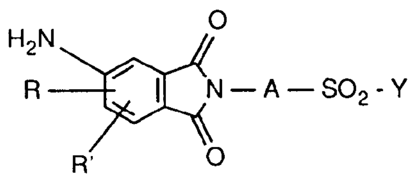

(12)

diazotiert und auf in etwa 1 Moläquivalent einer Kupplungskomponente der Formel

$K-(H)_s$ (13),

kuppelt, worin A, K, R, R', Y und s jeweils die im Anspruch 1 genannte Bedeutung haben.

**16.** Verwendung von Verbindungen der Formel (1) als faserreaktive Farbstoffe zum Färben oder Bedrucken von hydroxylgruppenhaltigen oder stickstoffhaltigen Fasermaterialien.

**17.** Verwendung gemäss Anspruch 16, dadurch gekennzeichnet, dass man cellulosehaltige Fasermaterialien, insbesondere baumwollhaltige Fasermaterialien, färbt oder bedruckt.

**18.** Verbindungsgemisch enthaltend je eine Verbindung der Formel

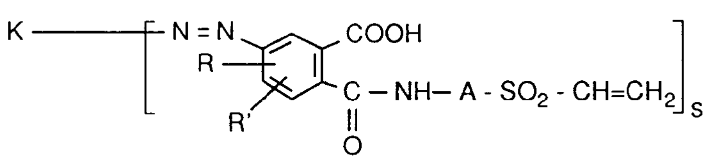

(16a) und

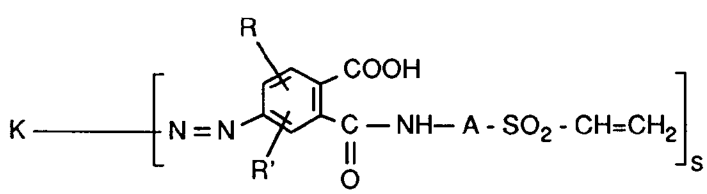

(16b),

worin A, K, R, R' und s jeweils die im Anspruch 1 angegebene Bedeutung haben.

**19.** Verfahren zur Herstellung eines Verbindungsgemisches enthaltend je eine Verbindung der Formel (16a) und (16b) gemäss Anspruch 18, dadurch gekennzeichnet, dass man eine Verbindung der Formel (1) gemäss Anspruch 1 in einem alkalischen Medium behandelt.

**20.** Verwendung eines Verbindungsgemisches enthaltend je eine Verbindung der Formel (16a) und (16b) gemäss Anspruch 18 als faserreaktive Farbstoffe zum Färben oder Bedrucken von hydroxylgruppenhaltigen oder stickstoffhaltigen Fasermaterialien.